(19) Europäisches Patentamt
European Patent Office
Office européen des brevets

(11) EP 4 292 647 B1

(12) **EUROPEAN PATENT SPECIFICATION**

(45) Date of publication and mention of the grant of the patent:
**06.05.2026 Bulletin 2026/19**

(21) Application number: **23178857.1**

(22) Date of filing: **13.06.2023**

(51) International Patent Classification (IPC):
***A61N 5/10*** *(2006.01)*

(52) Cooperative Patent Classification (CPC):
**A61N 5/1031**

(54) **PARALLEL PROCESSING FOR MULTI-PASS OPTIMIZATION OF RADIOTHERAPY PLANS**

PARALLELE VERARBEITUNG ZUR OPTIMIERUNG VON STRAHLENTHERAPIEPLÄNEN IN MEHREREN DURCHGÄNGEN

TRAITEMENT PARALLÈLE POUR OPTIMISATION MULTIPASSE DE PLANS DE RADIOTHÉRAPIE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **13.06.2022 US 202217806645**

(43) Date of publication of application:
**20.12.2023 Bulletin 2023/51**

(73) Proprietor: **Elekta Instrument AB**
**103 93 Stockholm (SE)**

(72) Inventors:
- **DA SILVA, Joakim Sebastian**
  **103 93 Stockholm (SE)**
- **HENNIX, Marcus Lars Eric**
  **103 93 Stockholm (SE)**
- **NORDSTRÖM, Carl Axel Håkan**
  **103 93 Stockholm (SE)**

(74) Representative: **Hamer, Thomas Daniel**
**Kilburn & Strode LLP**
**Lacon London**
**84 Theobalds Road**
**London WC1X 8NL (GB)**

(56) References cited:
**US-A1- 2021 158 929**

- **XINMIN LIU ET AL: "Constrained quadratic optimization for radiation treatment planning by use of graph form ADMM", 2016 AMERICAN CONTROL CONFERENCE (ACC), AMERICAN AUTOMATIC CONTROL COUNCIL (AACC), 6 July 2016 (2016-07-06), pages 5599 - 5604, XP032933564, DOI: 10.1109/ACC.2016.7526548**
- **HAO GAO: "Robust fluence map optimization via alternating direction method of multipliers with empirical parameter optimization", PHYSICS IN MEDICINE AND BIOLOGY, INSTITUTE OF PHYSICS PUBLISHING, BRISTOL GB, vol. 61, no. 7, 17 March 2016 (2016-03-17), pages 2838 - 2850, XP020301650, ISSN: 0031-9155, [retrieved on 20160317], DOI: 10.1088/0031-9155/61/7/2838**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 292 647 B1

## Description

TECHNICAL FIELD

Embodiments of the present disclosure pertain generally to processing and optimization techniques used in connection with a radiation therapy planning and treatment system. In particular, the present disclosure pertains to methods for the use of specific computing hardware configurations to optimize dosage in a radiation therapy session, which involve multiple passes or iterations to perform an optimization.

BACKGROUND

Radiation therapy (or "radiotherapy") can be used to treat cancers or other ailments in mammalian (e.g., human and animal) tissue. One such radiotherapy technique is provided using a Gamma Knife, by which a patient is irradiated by a large number of low-intensity gamma rays that converge with high intensity and high precision at a target (e.g., a tumor). Another such radiotherapy technique is provided using a linear accelerator (Linac), whereby a tumor is irradiated by high-energy particles (e.g., electrons, protons, ions, high-energy photons, and the like). The placement and dose of the radiation beam must be accurately controlled to ensure the tumor receives the prescribed radiation, and the placement of the beam should be such as to minimize damage to the surrounding healthy tissue, often called the organ(s) at risk (OARs).

In radiotherapy, treatment plans are usually generated by solving an optimization problem that balances various conflicting objectives, such as high dose to target, normal tissue sparing, and treatment complexity. It is therefore a multicriteria optimization (MCO) problem. Commonly, the different criteria are combined using a weighted sum, where each weight determines the relative importance of that criterion. Finding acceptable weights to develop an optimal radiotherapy plan with prior techniques may involve a manual and tedious process of trial-and-error, especially because evaluating a single choice of parameters (e.g., a single set of weight values) requires solving a full optimization problem for the radiotherapy treatment. Solving a full optimization problem for a single treatment plan (i.e. corresponding to a single set of weights) with conventional planning techniques may take from a few seconds up to an hour, depending on the application.

When performing a dose optimization process for multiple anatomical regions to be treated, two (or more) passes (i.e., two iterations) of processing operations may be needed. The first pass may be used to determine an optimal dose distribution corresponding to a set of weights, which is calculated based on a number of dose points selected from different volumes, such as a target volume (e.g., the designated target region to be treated with radiation, such as a tumor), a ring volume (e.g., a margin region around the target volume where a rapid fall-off of the radiation dose is desired to spare heathy tissue close to the target), a low-dose volume (e.g., a region surrounding the margin again, used as a proxy to limit the volume of healthy tissue further away from the target receiving at least a given fraction (e.g., half) of the prescription dose), and an organs at risk (OAR) volume (e.g., regions which are to be spared from radiation as much as possible). The approximate, geometric description of the low-dose volume used in the first pass is not sufficiently accurate to encompass the relevant region around where the optimized dose falls to below the given fraction of the prescription dose, for all targets and weight combinations. Thus, a second pass, where new low-dose points are selected from the low-dose volume obtained after the first pass, is necessary to achieve good control over the low-dose volume. This second pass requires additional computation time and processing resources. An exemplary two-pass optimization process is described in publication US 2021/158929 A1.

OVERVIEW

The invention is defined by the claims. Various embodiments, methods, systems, and computer-readable mediums are provided for the generation of radiotherapy plans, which provide an optimized process of calculation of radiotherapy dose distributions, using parallel processing computing hardware. This optimized process may be used for calculating dosage with the parallel processing computing hardware during multiple iterations or passes, where a first pass is used to compute an dose distribution corresponding to a set of weights, such that the first pass is calculated based on a first set of dose points, and where a second pass is used to calculate a more accurate plan based on new dose points obtained from the solution of produced by the first pass (such as new low-dose points selected from a "low-dose" region of the first optimization receiving a given fraction of the prescription dose).

The following enables the use of radiotherapy treatment planning optimization using a specialized configuration of a linear programming (LP) solver on parallel processing hardware, to optimize, in parallel, multiple treatment plans where the optimization procedure requires multiple iterations (also referred to as multiple passes). In an example, a LP solver is adapted to apply an alternating direction method of multipliers (ADMM) technique which enables execution of the LP solver on parallel processing hardware such as GPUs, accelerator devices, specialized parallel processor architectures (including CPUs in multi-core and multiprocessor configurations), massively parallel processors, and the like. The LP equations corresponding to multiple sets of weights can be solved in parallel, in a substantially faster manner than being solved sequentially. Additionally, modifications are made to the optimization results from a first pass, to allow the use of the LP solver and the ADMM technique on a second pass.

According to the invention, operations for multi-pass dose optimization for a radiotherapy treatment plan include: obtaining a set of first optimization problems for providing radiotherapy treatment to a human subject, the first optimization problems defined by a first plurality of parameters; performing dose optimization for delivery of the radiotherapy treatment to at least one treatment area of the human subject (e.g., a target area and a low-dose area), using two passes of the dose optimization; and generating treatment plan data based on at least one of the solutions produced by the second pass of the dose optimization, with such treatment plan data is used to control delivery of radiotherapy from a radiotherapy machine. The dose optimization includes: converting the first optimization problems into a first problem matrix; performing a first pass of the dose optimization by solving, using a linear programming solver operating based on an alternating direction method of multipliers technique, the first optimization problems (represented in the first problem matrix) in parallel on parallel processing hardware, the first pass to produce a first set of multiple solutions, corresponding to multiple sets of weights, to the first optimization problems; combining the first set of multiple solutions to the first optimization problems to produce a set of second optimization problems for providing the radiotherapy treatment, with the second optimization problems defined by a second plurality of parameters; converting the second optimization problems into a second problem matrix; and performing a second pass of the dose optimization by solving, using a linear programming solver operating based on an alternating direction method of multipliers technique, the second optimization problems (represented in the second problem matrix) in parallel on the parallel processing hardware, the second pass to produce multiple solutions, corresponding to multiple sets of weights, to the second optimization problems.

In further examples, the first and the second plurality of parameters, which establish properties for the first and second optimization problems, define constraints for at least one target area and at least one low-dose area in the at least one treatment area. The first plurality of parameters and the second plurality of parameters may also relate to radiation delivery parameters of a radiotherapy treatment machine. Further, the multiple sets of weights, corresponding to the first and second set of multiple solutions from the first pass or second pass, may relate to points (low-dose points) defined for at least one low-dose volume.

According to the invention, the at least one treatment area includes a low-dose region and a target region, such as where a dose to be delivered in the low-dose region is a fraction of a dose to be delivered in the target region. Accordingly, combining the first set of multiple solutions (e.g., to produce the second optimization problems) includes performing a union of the first set of multiple solutions to the first optimization problems for the low-dose region. Combining the first set of multiple solutions to produce the second optimization problems may also include performing a sampling of the union of the first set of multiple solutions to identify weights of the second plurality of parameters for the low-dose region. In one example, each low-dose point selected from a common low-dose region of the first set of multiple solutions of the first optimization problems is represented in the resulting second problem matrix, and performing the second pass of the dose optimization includes assigning a non-zero upper bound to a solution vector in a subset of points corresponding a respective low-dose region for each set of weights. In an alternative example, each low-dose point selected from a common low-dose region of the first set of multiple solutions of the first optimization problem is represented in the second problem matrix, and performing the second pass of the dose optimization includes applying a new low-dose weight to all low-dose points in the union of the first set of multiple solutions of the first optimization problems.

As noted, converting the linear programming equations may comprise applying an alternating direction method of multipliers (ADMM) technique. Specifically, the ADMM technique may comprise transforming the linear programming equations to matrix and projection operations (e.g., as described with reference to Equation 6, below). The parallel processing hardware may comprise a plurality of graphics processing units (GPUs), accelerator devices, specialized parallel processor configurations, massively parallel processors, and the like which can execute the matrix operations in parallel. In a specific example, solving the first optimization problems or the second optimization problems in parallel on the parallel processing hardware includes, for a respective set of problems, identifying parameterized linear programming equations from the respective set of problems, and converting the parameterized linear programming equations for execution by the parallel processing hardware. Further, solving the respective set of problems in parallel may include solving a plurality of the converted parameterized linear programming equations in parallel on the parallel processing hardware, to produce a plurality of solutions to the respective set of problems.

The results of the dose optimization may be used for identifying and developing a further solution that is implemented for radiotherapy treatment. For instance, this may include selecting a solution to the second optimization problems based on evaluation of the second set of multiple solutions, where the treatment plan data is generated based on the selected solution to the second optimization problem. For instance, the selected solution to the second optimization problems may provide an approximate solution, and be followed by receiving an additional optimization to the selected solution, such that the treatment plan data for the radiotherapy treatment is generated based on the additional optimization to the selected solution.

The treatment plan data for the radiotherapy treatment may comprise a set of treatment delivery parameters corresponding to capabilities of the radiotherapy treatment machine. In an example, the radiotherapy treatment is provided with a Gamma knife, and the set of treatment delivery parameters comprises a set of isocenters used for delivery of the radiotherapy treatment. For instance, the set of treatment delivery parameters further comprises timing for delivery of the radiotherapy treatment and a collimator sequence for the delivery of the radiotherapy treatment with the Gamma knife. As another example, the radiotherapy treatment is provided with a Volumetric-modulated arc therapy (VMAT) or Intensity modulated radiation therapy (IMRT), e.g., using a Linac radiotherapy machine, and the set of treatment delivery parameters comprises one or more of: a set of arc control points for one or more arcs, fluence fields, gantry speed, and dose rate along the one or more arcs.

In further examples, the operations may be followed by operations that cause or effect the delivery of the radiotherapy treatment using a plurality of radiotherapy beams from the radiotherapy treatment machine, based on the treatment plan data for the radiotherapy treatment.

The above overview is intended to provide an overview of subject matter of the present patent application. It is not intended to provide an exclusive or exhaustive explanation of the inventive subject matter. The detailed description is included to provide further information about the present patent application.

## BRIEF DESCRIPTION OF THE DRAWINGS

In the drawings, which are not necessarily drawn to scale, like numerals describe substantially similar components throughout the several views. Like numerals having different letter suffixes represent different instances of substantially similar components. The drawings illustrate generally, by way of example but not by way of limitation, various embodiments discussed in the present document.

**FIG. 1** illustrates a radiotherapy system, according to some examples.

**FIG. 2A** illustrates a radiotherapy system having output configured to provide a therapy beam, according to some examples.

**FIG. 2B** illustrates a system including a combined radiation therapy system and an imaging system, such as a cone beam computed tomography (CBCT) imaging system, according to some examples.

**FIG. 3** illustrates a partially cut-away view of a system including a combined radiation therapy system and an imaging system, such as a nuclear magnetic resonance (MR) imaging (MRI) system, according to some examples.

**FIG. 4** illustrates an example of a Leksell Gamma Knife radiotherapy device, according to some examples.

**FIG. 5** illustrates a radiotherapy treatment planning workflow using parallel processing for radiology problem optimization, according to some examples.

**FIG. 6** illustrates a workflow for performing multi-pass operations of radiology problem optimization with parallel processing, according to some examples.

**FIG. 7A** illustrates results from serial processing of multiple passes in radiology problem optimization, according to some examples.

**FIG. 7B** illustrates results from parallel processing of multiple passes in radiology problem optimization, according to some examples.

**FIG. 8** illustrates a flowchart for a method of radiotherapy treatment planning, according to some examples.

**FIG. 9** illustrates an exemplary block diagram of a machine on which one or more of the methods as discussed herein can be implemented.

## DETAILED DESCRIPTION

In the following detailed description, reference is made to the accompanying drawings which form a part hereof, and which is shown by way of illustration-specific embodiments in which the present disclosure may be practiced. These embodiments, which are also referred to herein as "examples," are described in sufficient detail to enable those skilled in the art to practice the disclosure, and it is to be understood that the embodiments may be combined, or that other embodiments may be utilized and that structural, logical and electrical changes may be made. The invention is defined by the appended claims.

The following discusses various implementations of planning and optimization techniques usable in radiotherapy or radiosurgery applications. Such techniques may be used to generate solutions to a radiotherapy problem, which may correspond to different parameter choices and weight values, and generate treatment plan data based on at least one of the found solutions. Such techniques may further be used to provide an optimized second pass or iteration, to further refine the shape and size of the low-dose region by selecting low-dose points for use in the second pass based on the low-dose volume estimated from the result of the first optimization pass. The radiotherapy problems that are analyzed in each of the passes may be solved in parallel with the use of parallel processing hardware, to efficiently and effectively produce multiple solutions for the radiotherapy problem.

As used herein, a "point" refers to the point at which the radiotherapy dose is evaluated in the optimization. With the use of the low-dose volume, the optimization objective is to keep the dose in the target points at or above a prescription dose, the dose at the ring points below the prescription dose, and the dose at the low-dose points below a fraction of the prescription dose. A low-dose point is selected from the region close to the boundary of the volume obtaining a given fraction of the prescription dose in order to control this region in the optimization. However, as is understood, it is difficult to guess/find a good geometric representation for such a region. Therefore, the first pass is run; based on the result (e.g. the dose distribution) from the first pass; more appropriate low-dose points are selected; and then, the problem is solved again using these selected low-dose points in the second pass.

In the examples discussed herein, treatment planning problems are solved through use of a LP solver for a problem matrix which operates based on the alternating direction method of multipliers (ADMM). This LP solver is able to solve a large number of problems in parallel, provided that they share the same problem matrix, using parallel processing hardware such as graphical processing units (GPUs accelerator devices, specialized parallel processor configurations, etc). The use of ADMM and parallel processing execution approaches can (approximately) solve all the LPs sharing the same problem matrix but having different values of the optimization weights in parallel (e.g. simultaneously or substantially simultaneously), which is substantially faster than solving the LPs sequentially. The approaches for using an LP solver for radiotherapy plan optimization may be an adaption of those discussed in Patent Application No. PCT/EP2022/053438, publication WO 2023/151816 A1, titled PARALLEL GENERATION OF PARETO OPTIMAL RADIOTHERAPY PLANS, filed February 11, 2022.

The multi-pass optimization, discussed herein, results in the generation of a plurality of solutions which are used to identify set of a parameter choices for a clinically satisfactory radiotherapy plan. As will be understood, the radiotherapy problem may be expressed as a multicriteria optimization (MCO) problem, where the parameters correspond to clinical preferences. In a radiotherapy planning setting (e.g., planning for a Gamma Knife treatment), a sufficiently accurate approximation of optimal plans and regions within these plans may be generated with the present techniques much faster than conventional approaches. Specifically, the following techniques enable multiple-pass optimization, even in scenarios where an entire Pareto surface of Pareto-optimal treatment plans are identified and optimized for use with radiotherapy planning. A Pareto-optimal plan, as used herein, refers to a plan which is optimized such that no criterion can be improved without worsening another. The set of all Pareto-optimal plans constitutes the Pareto surface (also known as the Pareto frontier).

Significant technical and clinical benefits may be achieved by transforming a multi-pass radiotherapy plan optimization into respective LP problems, and solving the respective LP problems within a parallel processing space. The technical benefits include reduced computing processing times to generate output (e.g., radiotherapy treatment plans), enhanced computation solutions for achieving radiotherapy plan optimization, and accompanying improvements in processing, memory, and network resources used to generate radiotherapy treatments. The clinical benefits include faster computation, evaluation, and optimization times when the patient is awaiting treatment, and potentially more treatment options being presented to a clinician, allowing more precise and customized radiotherapy treatments to be generated and implemented for a particular patient.

Prior approaches for pre-calculating a representative set of treatment plans with conventional processing techniques often resulted in significant computation time and delays. The number of representative plans needed to span a Pareto surface for a Gamma Knife treatment is typically on the order of hundreds or thousands of plans, which means that a very long computation time (typically an overnight run) is required to generate a full representation of possible solutions. This computation time is further complicated by the need to perform a second pass on the possible solutions when attempting to optimize regions with different treatment dosages, such as "low-dose" regions designated to penalize regions receiving more dose than some fraction of the prescription dose (e.g., more than half the prescription dose, $V_{DP/2}$). Some prior approaches have attempted to develop AI-based methods for producing estimated solutions to these types of radiotherapy optimization problems. However, AI-based methods require large amounts of training data to cover all edge cases, and often require integration with a larger AI set of tools to be deployed in a product.

These and other limitations are addressed in the following configurations. The following paragraphs provide an overview of example radiotherapy system implementations and treatment use cases (with reference to **FIGS. 2A, 2B, 3** and **4),** including with the use of computing systems and hardware implementations (with reference to **FIGS. 1** and **9).** The following then continues with a discussion of example treatment planning and algorithm processing workflows (with reference to **FIGS. 5** and **6).** This is followed by a discussion of multi-pass radiotherapy optimization (with reference to **FIGS. 7A** and **7B),** as applied within parallel processing hardware. Finally, a discussion of radiotherapy treatment planning (with reference to **FIG. 8)** is provided, which illustrates an end-to-end method of generating an optimized treatment plan.

**FIG. 1** illustrates an exemplary radiotherapy system 100 adapted to perform radiotherapy plan processing operations using one or more of the approaches discussed herein. These radiotherapy plan processing operations are performed to enable the radiotherapy system 100 to provide radiation therapy to a patient based on specific aspects of captured medical imaging data and therapy dose calculations or radiotherapy machine configuration parameters. Specifically, the following processing operations may be implemented as part of the radiotherapy planning logic 120 for developing a radiotherapy treatment plan. It will be understood, however, that many variations and use cases of the following planning logic 120 and optimization operations may be provided, such as in response to data verification, visualization, and other medical evaluative and diagnostic operations.

The radiotherapy system 100 includes a radiotherapy processing computing system 110 which hosts radiotherapy planning logic 120. The radiotherapy processing computing system 110 may be connected to a network (not shown), and such network may be connected to the Internet. For instance, a network can connect the radiotherapy processing computing system 110 with one or more private and/or public medical information sources (e.g., a radiology information system (RIS), a medical record system (e.g., an electronic medical record (EMR)/ electronic health record (EHR) system), an oncology information system (OIS)), one or more image data sources 150, an image acquisition device 170 (e.g., an imaging modality), a treatment device 180 (e.g., a radiation therapy device), and a treatment data source 160.

As an example, the radiotherapy processing computing system 110 can be configured to receive a treatment goal of a subject (e.g., from one or more MR images) and generate a radiotherapy treatment plan by executing instructions or data from the radiotherapy planning logic 120, as part of operations to generate treatment plans to be used by the treatment device 180 and/or for output on device 146. In an embodiment, the radiotherapy planning logic 120 solves an optimization problem to generate the radiotherapy treatment plan. The radiotherapy planning logic 120 solves the radiotherapy optimization problem by estimating optimization variables of the received optimization problem. Then, the optimization problem is solved using an optimization problem solver. Such optimization problem solvers include, e.g., a simplex method, an interior point method, a Newton method, a quasi-Newton method, a Gauss-Newton method, a Levenberg-Marquardt method, a linear least-squares method, a gradient descent method, a projected gradient method, a conjugate gradient method, an augmented Lagrangian method, a Nelder-Mead method, a branch and bound method, a cutting plane method, simulated annealing, and/or sequential quadratic programming, or as discussed below, the use of ADMM applied on parallel processing circuitry 118.

A generic radiotherapy treatment plan optimization problem can be defined as Equation 1:

$$\underset{x \in X}{\text{minimize}}\, f(x)$$
$$\text{subject to}\; x \in \Omega$$

**(EQUATION 1)**

where $f: X \to \mathbb{R}$ is an objective function, $x \in X$ are the decision variables (also referred to as optimization variables) and $\Omega \subseteq X$ is the set of feasible variables. In general, the function $f$ can be nonlinear and the set $\Omega$ non-convex. The optimization problems are typically solved using some form of iterative scheme. For example, in case $f$ is smooth and convex, and $\Omega$ is convex, then the projected gradient scheme can be used to solve equation (1) and reads as follows:

$$x_{n+1} = \text{proj}_{\,\Omega}(x_n - \eta \nabla f(x_n))$$

**(EQUATION 2)**

where $\text{proj}_{\Omega}: X \to X$ is the projection onto $\Omega$, $\eta \in \mathbb{R}$ is a stepsize and $\nabla f: X \to X$ the gradient. While these algorithms are typically provably convergent (e.g., given enough time (and correct parameter choices), the algorithm will converge to a minimizer), they are not always very fast and efficient. In fact, several algorithms may require hundreds if not thousands of iterations in order to achieve approximate convergence. Since each step may be computationally expensive, this may imply runtimes of minutes or even hours.

Solutions to such optimization problems can be produced by applying solver methods to solve the optimization problems with use of parallel processing hardware, including for the optimization of multiple regions (including a low-dose region) using multiple passes. As used herein, a "region" is interchangeable or synonymous with an "area" or "volume", when referring to the three-dimensional space from which dose points are sampled. In some scenarios, the optimization problems are solved within a deviation threshold of a desired or expected solution. The disclosed techniques enhance the speed and efficiency of solving the optimization problem by the parallel execution of the solver methods, including for the optimization of the multiple regions with multiple passes. As will be understood, methods used to solve an optimization problem will often apply an accuracy exit criteria (deviation threshold). However, the use of ADMM as discussed herein, in addition to being easily parallelizable, will quickly produce a reasonably accurate solution. Thus, the solution produced by ADMM is likely to provide information that can be used to make a decision whether the solution is of clinical interest or not.

The radiotherapy processing computing system 110 may include processing circuitry 112, memory 114, a storage device 116, parallel processing circuitry 118, and other hardware and software-operable features such as a user interface 142, a communication interface (not shown), and the like. The storage device 116 may store transitory or non-transitory computer-executable instructions, such as an operating system, radiation therapy treatment plans, training data, software programs (e.g., image processing software, image or anatomical visualization software, artificial intelligence (AI) or ML implementations and algorithms such as provided by deep learning models, ML models, and neural networks (NNs), etc.), and any other computer-executable instructions to be executed by the processing circuitry 112.

In an example, the processing circuitry 112 may include at least one processing device, such as one or more general-purpose processing devices such as a microprocessor, a central processing unit (CPU), a graphics processing unit (GPU), an accelerated processing unit (APU), or the like. More particularly, the processing circuitry 112 may be a complex instruction set computing (CISC) microprocessor, a reduced instruction set computing (RISC) microprocessor, a very long instruction Word (VLIW) microprocessor, a processor implementing other instruction sets, or processors implementing a combination of instruction sets. The processing circuitry 112 may also be implemented by one or more special-purpose processing devices such as an application-specific integrated circuit (ASIC), a field programmable gate array (FPGA), a digital signal processor (DSP), a System on a Chip (SoC), or the like.

As would be appreciated by those skilled in the art, in some examples, the processing circuitry 112 may be a special-purpose processor rather than a general-purpose processor. The processing circuitry 112 may include one or more known processing devices, such as a microprocessor from the Pentium™, Core™, Xeon™, or Itanium™ family manufactured by Intel®, the Turion™, Athlon™, Sempron™, Opteron™, FX™, Phenom™ family manufactured by AMD™, or any of various processors manufactured by Sun Microsystems. The processing circuitry 112 may also include graphical processing units such as a GPU device provided from the GeForce®, Quadro®, Tesla® family manufactured by Nvidia™, GMA, Arc™ family manufactured by Intel®, or the Radeon™ family manufactured by AMD®. The processing circuitry 112 may also include accelerated processing units such as the Xeon™ family manufactured by Intel®. The disclosed embodiments are not limited to any type of processor(s) otherwise configured to meet the computing demands of identifying, analyzing, maintaining, generating, and/or providing large amounts of data or manipulating such data to perform the methods disclosed herein. In addition, the term "processor" may include more than one physical (circuitry-based) or software-based processor (for example, a multi-core design or a plurality of processors each having a multi-core design). The processing circuitry 112 can execute sequences of transitory or non-transitory computer program instructions, stored in memory 114, and accessed from the storage device 116, to perform various operations, processes, and methods that will be explained in greater detail below. It should be understood that any component in system 100 may be implemented separately and operate as an independent device and may be coupled to any other component in system 100 to perform the techniques described in this disclosure.

The memory 114 may comprise read-only memory (ROM), a phase-change random access memory (PRAM), a static random access memory (SRAM), a flash memory, a random access memory (RAM), a dynamic random access memory (DRAM) such as synchronous DRAM (SDRAM), an electrically erasable programmable read-only memory (EEPROM), a static memory (e.g., flash memory, flash disk, static random access memory) as well as other types of random access memories, a cache, a register, a compact disc read-only memory (CD-ROM), a digital versatile disc (DVD) or other optical storage, a cassette tape, other magnetic storage device, or any other non-transitory medium that may be used to store information including images, training data, one or more ML model(s) or technique(s) parameters, data, or transitory or non-transitory computer executable instructions (e.g., stored in any format) capable of being accessed by the processing circuitry 112, or any other type of computer device. For instance, the computer program instructions can be accessed by the processing circuitry 112, read from the ROM, or any other suitable memory location, and loaded into the RAM for execution by the processing circuitry 112.

The storage device 116 may constitute a drive unit that includes a transitory or non-transitory machine-readable medium on which is stored one or more sets of transitory or non-transitory instructions and data structures (e.g., software) embodying or utilized by any one or more of the methodologies or functions described herein (including, in various examples, the radiotherapy planning logic 120 and the user interface 142). The instructions may also reside, completely or at least partially, within the memory 114 and/or within the processing circuitry 112 during execution thereof by the radiotherapy processing computing system 110, with the memory 114 and the processing circuitry 112 also constituting transitory or non-transitory machine-readable media. The instructions may also cause the parallel processing circuitry 118 to perform specific processing operations.

The memory 114 and the storage device 116 may constitute a non-transitory computer-readable medium. For example, the memory 114 and the storage device 116 may store or load transitory or non-transitory instructions for one or more software applications on the computer-readable medium. Software applications stored or loaded with the memory 114 and the storage device 116 may include, for example, an operating system for common computer systems as well as for software-controlled devices. The radiotherapy processing computing system 110 may also operate a variety of software programs comprising software code for implementing the radiotherapy planning logic 120 and the user interface 142. Further, the memory 114 and the storage device 116 may store or load an entire software application, part of a software application, or code or data that is associated with a software application, which is executable by the processing circuitry 112. In a further example, the memory 114 and the storage device 116 may store, load, and manipulate one or more radiation therapy treatment plans, imaging data, segmentation data, treatment visualizations, histograms or measurements, one or more AI model data (e.g., weights and parameters of one or more ML model(s)), training data, labels and mapping data, and the like. It is contemplated that software programs may be stored not only on the storage device 116 and the memory 114 but also on a removable computer medium, such as a hard drive, a computer disk, a CD-ROM, a DVD, a Blu-Ray DVD, USB flash drive, a SD card, a memory stick, or any other suitable medium; such software programs may also be communicated or received over a network.

The parallel processing circuitry 118 may include any of the processing circuitry described above arranged into a parallel processing configuration. For instance, a set of graphical processing units (e.g., GPUs from the GeForce®, Quadro®, Tesla® family manufactured by Nvidia®, GMA, Arc™ family manufactured by Intel®, or the Radeon™ family manufactured by AMD®) may be arranged to perform highly parallel or repetitive computing tasks simultaneously. As used herein, a set of graphical processing units may refer to a single GPU device (e.g., card) having a single GPU core or unit or a plurality of GPU cores or units, or multiple GPU devices (e.g., cards) with each device having single GPU core or unit or a plurality of GPU cores or units. Other specialized parallel processing units or hardware capable of performing multiple calculations simultaneously may also be deployed, including specialized CPU architectures and configurations. GPUs may include a single GPU "device" or "system" which operates or orchestrates numerous (e.g., tens, hundreds, or thousands) of sub-processors; many of the examples provided herein refer to the use of a single GPU device or system which uses each of its numerous sub-processors to process a respective set of parameters.

Although not depicted, the radiotherapy processing computing system 110 may include a communication interface, network interface card, and communications circuitry. An example communication interface may include, for example, a network adaptor, a cable connector, a serial connector, a USB connector, a parallel connector, a high-speed data transmission adaptor (e.g., such as fiber, USB 3.0, thunderbolt, and the like), a wireless network adaptor (e.g., such as an IEEE 802.11/Wi-Fi adapter), a telecommunication adapter (e.g., to communicate with 3G, 4G/LTE, and 5G networks and the like), and the like. Such a communication interface may include one or more digital and/or analog communication devices that permit a machine to communicate with other machines and devices, such as remotely located components, via a network. The network may provide the functionality of a local area network (LAN), a wireless network, a cloud computing environment (e.g., software as a service, platform as a service, infrastructure as a service, etc.), a client-server, a wide area network (WAN), and the like. For example, the network may be a LAN or a WAN that may include other systems (including additional image processing computing systems or image-based components associated with medical imaging or radiotherapy operations).

In an example, the radiotherapy processing computing system 110 may obtain image data 152 from the image data source 150 (e.g., MR images) for hosting on the storage device 116 and the memory 114. In yet another example, the software programs may substitute functions of the patient images such as signed distance functions or processed versions of the images that emphasize some aspect of the image information. The radiotherapy processing computing system 110 may obtain or communicate image data 152 from or to image data source 150. In further examples, the treatment data source 160 receives or updates the planning data 162 as a result of a treatment plan generated by the radiotherapy planning logic 120. The image data source 150 may also provide or host the imaging data for use in the radiotherapy planning logic 120.

In an example, computing system 110 may communicate with treatment data source(s) 160, input device 148, and other data sources to generate optimization variables and parameters for a plurality of radiotherapy treatment plan optimization problems. Such optimization variables and parameters are generated to identify a plurality of solutions to the radiotherapy problem. These solutions may approximate a solution, and may be further evaluated and refined before use (e.g., with additional optimization) in a radiotherapy treatment.

The processing circuitry 112 and the parallel processing circuitry 118 may be communicatively coupled to the memory 114 and the storage device 116, and the processing circuitry 112 and the parallel processing circuitry 118 may be configured to execute computer-executable instructions stored thereon from either the memory 114 or the storage device 116. Particularly, radiotherapy planning logic 120 receives an optimization problem that is derived from parameters for radiotherapy treatment. The processing circuitry 112 and parallel processing circuitry 118 may utilize software programs or implementations to optimize a radiotherapy dose for delivery to a patient, as part of developing an optimized solution to a radiotherapy problem as discussed herein. Further, such software programs or implementations may utilize the radiotherapy planning logic 120 to produce new or updated treatment plan parameters for deployment to the treatment data source 160 and/or presentation on output device 146, using the techniques further discussed herein. The processing circuitry 112 or the parallel processing circuitry 118 may subsequently then transmit the new or updated treatment plan details via a communication interface and the network to the treatment device 180, where the radiation therapy plan will be used to treat a patient with radiation via the treatment device 180, consistent with results of the radiotherapy planning logic 120 (e.g., according to the processes discussed below).

In an example, the image data 152 used for defining a radiotherapy problem or indicating the anatomical regions of the patient may include one or more MRI image (e.g., 2D MRI, 3D MRI, 2D streaming MRI, 4D MRI, 4D volumetric MRI, 4D cine MRI, etc.), functional MRI images (e.g., fMRI, DCE-MRI, diffusion MRI), Computed Tomography (CT) images (e.g., 2D CT, 2D Cone beam CT, 3D CT, 3D CBCT, 4D CT, 4DCBCT), ultrasound images (e.g., 2D ultrasound, 3D ultrasound, 4D ultrasound), Positron Emission Tomography (PET) images, X-ray images, fluoroscopic images, radiotherapy portal images, Single-Photo Emission Computed Tomography (SPECT) images, computer-generated synthetic images (e.g., pseudo-CT images) and the like. Further, the image data 152 may also include or be associated with medical image processing data (for example, training images, ground truth images, contoured images, and dose images). In other examples, an equivalent representation of an anatomical region may be represented in non-image formats (e.g., coordinates, mappings, etc.).

In an example, the image data 152 may be received from the image acquisition device 170 and stored in one or more of the image data sources 150 (e.g., a Picture Archiving and Communication System (PACS), a Vendor Neutral Archive (VNA), a medical record or information system, a data warehouse, etc.). Accordingly, the image acquisition device 170 may comprise an MRI imaging device, a CT imaging device, a PET imaging device, an ultrasound imaging device, a fluoroscopic device, a SPECT imaging device, an integrated Linear Accelerator and MRI imaging device, CBCT imaging device, or other medical imaging devices for obtaining the medical images of the patient. The image data 152 may be received and stored in any type of data or any type of format (e.g., in a Digital Imaging and Communications in Medicine (DICOM) format) that the image acquisition device 170 and the radiotherapy processing computing system 110 may use to perform operations consistent with the disclosed embodiments. Further, in some examples, the models discussed herein may be trained to process the original image data format or a derivation thereof.

In an example, the image acquisition device 170 may be integrated with the treatment device 180 as a single apparatus (e.g., an MRI device combined with a linear accelerator, also referred to as an "MRI-Linac"). Such an MRI-Linac can be used, for example, to determine a location of a target organ or a target tumor in the patient so as to direct radiation therapy accurately according to the radiation therapy treatment plan to a predetermined target. For instance, a radiation therapy treatment plan may provide information about a particular radiation dose to be applied to each patient. The radiation therapy treatment plan may also include other radiotherapy information, including control points of a radiotherapy treatment device, such as couch position, beam intensity, beam angles, dose-histogram-volume information, the number of radiation beams to be used during therapy, the dose per beam, and the like.

The radiotherapy processing computing system 110 may communicate with an external database through a network to send/receive a plurality of various types of data related to image processing and radiotherapy operations. For example, an external database may include machine data (including device constraints) that provides information associated with the treatment device 180, the image acquisition device 170, or other machines relevant to radiotherapy or medical procedures. Machine data information (e.g., control points) may include radiation beam size, arc placement, beam on and off time duration, machine parameters, segments, multi-leaf collimator (MLC) configuration, gantry speed, MRI pulse sequence, and the like. The external database may be a storage device and may be equipped with appropriate database administration software programs. Further, such databases or data sources may include a plurality of devices or systems located either in a central or a distributed manner.

The radiotherapy processing computing system 110 can collect and obtain data, and communicate with other systems, via a network using one or more communication interfaces, which are communicatively coupled to the processing circuitry 112 and the memory 114. For instance, a communication interface may provide communication connections between the radiotherapy processing computing system 110 and radiotherapy system components (e.g., permitting the exchange of data with external devices). For instance, the communication interface may, in some examples, have appropriate interfacing circuitry from an output device 146 or an input device 148 to connect to the user interface 142, which may be a hardware keyboard, a keypad, or a touch screen through which a user may input information into the radiotherapy system.

As an example, the output device 146 may include a display device that outputs a representation of the user interface 142 and one or more aspects, visualizations, or representations of the medical images, the treatment plans, and statuses of training, generation, verification, or implementation of such plans. The output device 146 may include one or more display screens that display medical images, interface information, treatment planning parameters (e.g., contours, dosages, beam angles, labels, maps, etc.), treatment plans, a target, localizing a target and/or tracking a target, or any related information to the user. The input device 148 connected to the user interface 142 may be a keyboard, a keypad, a touch screen or any type of device that a user may use to the radiotherapy system 100. Alternatively, the output device 146, the input device 148, and features of the user interface 142 may be integrated into a single device such as a smartphone or tablet computer (e.g., Apple iPad®, Lenovo Thinkpad®, Samsung Galaxy®, etc.).

Furthermore, any and all components of the radiotherapy system may be implemented as a virtual machine (e.g., via VMWare, Hyper-V, and the like virtualization platforms) or independent devices. For instance, a virtual machine can be software that functions as hardware. Therefore, a virtual machine can include at least one or more virtual processors, one or more virtual memories, and one or more virtual communication interfaces that together function as hardware. For example, the radiotherapy processing computing system 110, the image data sources 150, or like components, may be implemented as a virtual machine or within a cloud-based virtualization environment.

The image acquisition device 170 can be configured to acquire one or more images of the patient's anatomy for a region of interest (e.g., a target organ, a target tumor or both). Each image, typically a 2D image or slice, can include one or more parameters (e.g., a 2D slice thickness, an orientation, and a location, etc.). In an example, the image acquisition device 170 can acquire a 2D slice in any orientation. For example, an orientation of the 2D slice can include a sagittal orientation, a coronal orientation, or an axial orientation. The processing circuitry 112 can adjust one or more parameters, such as the thickness and/or orientation of the 2D slice, to include the target organ and/or target tumor. In an example, 2D slices can be determined from information such as a 3D CBCT or CT or MRI volume. Such 2D slices can be acquired by the image acquisition device 170 in "near real time" while a patient is undergoing radiation therapy treatment (for example, when using the treatment device 180 (with "near real time" meaning acquiring the data in at least milliseconds or less)).

The radiotherapy planning logic 120 in the radiotherapy processing computing system 110 implements a radiotherapy optimization workflow 130 and treatment plan generation workflow 140. The radiotherapy optimization workflow 130 may implement optimization operations for identifying and developing radiotherapy plans, while the treatment plan generation workflow may implement operations for evaluating, selecting, and refining one of the radiotherapy plans. In specific examples, the radiotherapy optimization workflow 130 performs radiotherapy problem processing 132 to obtain and identify an optimization problem, problem conversion processing 134 to convert optimization problems for more effective or efficient execution on hardware (such as converting the optimization problems for execution with the parallel processing circuitry 118), problem optimization processing 136 to optimize the radiation dose distribution according to a given set of importance weights where each weight is associated with different regions (including a low-dose region), and solution processing 138 to identify and output solutions to the optimization problems. More details of the radiotherapy optimization workflow 130 are provided below with reference to FIGS. 5 and 6, including with the use of an alternating direction method of multipliers to perform multi-pass execution of a problem matrix on the parallel processing circuitry 118. Likewise, more details of the treatment plan generation workflow 140 are provided below with reference to FIGS. 6, 7B, and 8, which indicate how a treatment plan may be further evaluated, selected, and optimized, with multi-pass optimization operations.

**FIG. 2A** illustrates a radiation therapy device 202 that may include a radiation source, such as an X-ray source or a linear accelerator, a couch 216, an imaging detector 214, and a radiation therapy output 204. The radiation therapy device 202 may be configured to emit a radiation beam 208 to provide therapy to a patient. The radiation therapy output 204 can include one or more attenuators or collimators, such as an MLC. An MLC may be used for shaping, directing, or modulating an intensity of a radiation therapy beam to the specified target locus within the patient. The leaves of the MLC, for instance, can be automatically positioned to define an aperture approximating a tumor cross-section or projection, and cause modulation of the radiation therapy beam. For example, the leaves can include metallic plates, such as comprising tungsten, with a long axis of the plates oriented parallel to a beam direction and having ends oriented orthogonally to the beam direction. Further, a "state" of the MLC can be adjusted adaptively during a course of radiation therapy treatment, such as to establish a therapy beam that better approximates a shape or location of the tumor or other target locus.

Referring back to FIG. 2A, a patient can be positioned in a region 212 and supported by the treatment couch 216 to receive a radiation therapy dose, according to a radiation therapy treatment plan. The radiation therapy output 204 can be mounted or attached to a gantry 206 or other mechanical support. One or more chassis motors (not shown) may rotate the gantry 206 and the radiation therapy output 204 around couch 216 when the couch 216 is inserted into the treatment area. In an example, gantry 206 may be continuously rotatable around couch 216 when the couch 216 is inserted into the treatment area. In another example, gantry 206 may rotate to a predetermined position when the couch 216 is inserted into the treatment area. For example, the gantry 206 can be configured to rotate the therapy output 204 around an axis ("*A*"). Both the couch 216 and the radiation therapy output 204 can be independently moveable to other positions around the patient, such as moveable in transverse direction ("*T*"), moveable in a lateral direction ("*L*"), or as rotation about one or more other axes, such as rotation about a transverse axis (indicated as "*R*"). A controller communicatively connected to one or more actuators (not shown) may control the couch 216 movements or rotations in order to properly position the patient in or out of the radiation beam 208 according to a radiation therapy treatment plan. Both the couch 216 and the gantry 206 are independently moveable from one another in multiple degrees of freedom, which allows the patient to be positioned such that the radiation beam 208 can target the tumor precisely. The MLC may be integrated and included within gantry 206 to deliver the radiation beam 208 of a certain shape.

The coordinate system (including axes *A, T,* and *L*) shown in FIG. 2A can have an origin located at an isocenter 210. The isocenter can be defined as a location where the central axis of the radiation beam 208 intersects the origin of a coordinate axis, such as to deliver a prescribed radiation dose to a location on or within a patient. Alternatively, the isocenter 210 can be defined as a location where the central axis of the radiation beam 208 intersects the patient for various rotational positions of the radiation therapy output 204 as positioned by the gantry 206 around the axis *A.* As discussed herein, the gantry angle corresponds to the position of gantry 206 relative to axis *A,* although any other axis or combination of axes can be referenced and used to determine the gantry angle.

Gantry 206 may also have an attached imaging detector 214. The imaging detector 214 is preferably located opposite to the radiation source, and in an example, the imaging detector 214 can be located within a field of the radiation beam 208. The imaging detector 214 can be mounted on the gantry 206 (preferably opposite the radiation therapy output 204), such as to maintain alignment with the radiation beam 208. The imaging detector 214 rotates about the rotational axis as the gantry 206 rotates. In an example, the imaging detector 214 can be a flat panel detector (e.g., a direct detector or a scintillator detector). In this manner, the imaging detector 214 can be used to monitor the radiation beam 208 or the imaging detector 214 can be used for imaging the patient's anatomy, such as portal imaging. The control circuitry of the radiation therapy device 202 may be integrated within the radiotherapy system 100 or remote from it.

In an illustrative example, one or more of the couch 216, the therapy output 204, or the gantry 206 can be automatically positioned, and the therapy output 204 can establish the radiation beam 208 according to a specified dose for a particular therapy delivery instance. A sequence of therapy deliveries can be specified according to a radiation therapy treatment plan, such as using one or more different orientations or locations of the gantry 206, couch 216, or therapy output 204. The therapy deliveries can occur sequentially, but can intersect in a desired therapy locus on or within the patient, such as at the isocenter 210. A prescribed cumulative dose of radiation therapy can thereby be delivered to the therapy locus while damage to tissue near the therapy locus can be reduced or avoided.

**FIG. 2B** illustrates a radiation therapy device 202 that may include a combined Linac and an imaging system, such as a CT imaging system. The radiation therapy device 202 can include an MLC (not shown). The CT imaging system can include an imaging X-ray source 218, such as providing X-ray energy in a kiloelectron-Volt (keV) energy range. The imaging X-ray source 218 can provide a fan-shaped and/or a conical radiation beam 208 directed to an imaging detector 222, such as a flat panel detector. The radiation therapy device 202 can be similar to the system described in relation to FIG. 2A, such as including a radiation therapy output 204, a gantry 206, a couch 216, and another imaging detector 214 (such as a flat panel detector). The X-ray source 218 can provide a comparatively-lower-energy X-ray diagnostic beam, for imaging.

In the illustrative example of FIG. 2B, the radiation therapy output 204 and the X-ray source 218 can be mounted on the same rotating gantry 206, rotationally separated from each other by 90 degrees. In another example, two or more X-ray sources can be mounted along the circumference of the gantry 206, such as each having its own detector arrangement to provide multiple angles of diagnostic imaging concurrently. Similarly, multiple radiation therapy outputs 204 can be provided.

**FIG. 3** depicts a radiation therapy system 300 that can include combining a radiation therapy device 202 and an imaging system, such as a magnetic resonance (MR) imaging system (e.g., known in the art as an MR-LINAC) consistent with the disclosed examples. As shown, system 300 may include a couch 216, an image acquisition device 320, and a radiation delivery device 330. System 300 delivers radiation therapy to a patient in accordance with a radiotherapy treatment plan. In some examples, image acquisition device 320 may correspond to image acquisition device 170 in FIG. 1 that may acquire origin images of a first modality (e.g., an MRI image) or destination images of a second modality (e.g., an CT image).

Couch 216 may support a patient (not shown) during a treatment session. In some implementations, couch 216 may move along a horizontal translation axis (labelled "I"), such that couch 216 can move the patient resting on couch 216 into and/or out of system 300. Couch 216 may also rotate around a central vertical axis of rotation, transverse to the translation axis. To allow such movement or rotation, couch 216 may have motors (not shown) enabling the couch 216 to move in various directions and to rotate along various axes. A controller (not shown) may control these movements or rotations in order to properly position the patient according to a treatment plan.

In some examples, image acquisition device 320 may include an MRI machine used to acquire 2D or 3D MRI images of the patient before, during, and/or after a treatment session. Image acquisition device 320 may include a magnet 321 for generating a primary magnetic field for magnetic resonance imaging. The magnetic field lines generated by operation of magnet 321 may run substantially parallel to the central translation axis I. Magnet 321 may include one or more coils with an axis that runs parallel to the translation axis I. In some examples, the one or more coils in magnet 321 may be spaced such that a central window 323 of magnet 321 is free of coils. In other examples, the coils in magnet 321 may be thin enough or of a reduced density such that they are substantially transparent to radiation of the wavelength generated by radiotherapy device 330. Image acquisition device 320 may also include one or more shielding coils, which may generate a magnetic field outside magnet 321 of approximately equal magnitude and opposite polarity in order to cancel or reduce any magnetic field outside of magnet 321. As described below, radiation source 331 of radiation delivery device 330 may be positioned in the region where the magnetic field is cancelled, at least to a first order, or reduced.

Image acquisition device 320 may also include two gradient coils 325 and 326, which may generate a gradient magnetic field that is superposed on the primary magnetic field. Coils 325 and 326 may generate a gradient in the resultant magnetic field that allows spatial encoding of the protons so that their position can be determined. Gradient coils 325 and 326 may be positioned around a common central axis with the magnet 321 and may be displaced along that central axis. The displacement may create a gap, or window, between coils 325 and 326. In examples where magnet 321 can also include a central window 323 between coils, the two windows may be aligned with each other.

In some examples, image acquisition device 320 may be an imaging device other than an MRI, such as an X-ray, a CT, a CBCT, a spiral CT, a PET, a SPECT, an optical tomography, a fluorescence imaging, ultrasound imaging, radiotherapy portal imaging device, or the like. As would be recognized by one of ordinary skill in the art, the above description of image acquisition device 320 concerns certain examples and is not intended to be limiting.

Radiation delivery device 330 may include the radiation source 331, such as an X-ray source or a Linac, and an MLC 332. Radiation delivery device 330 may be mounted on a chassis 335. One or more chassis motors (not shown) may rotate the chassis 335 around the couch 216 when the couch 216 is inserted into the treatment area. In an example, the chassis 335 may be continuously rotatable around the couch 216, when the couch 216 is inserted into the treatment area. Chassis 335 may also have an attached radiation detector (not shown), preferably located opposite to radiation source 331 and with the rotational axis of the chassis 335 positioned between the radiation source 331 and the detector. Further, the device 330 may include control circuitry (not shown) used to control, for example, one or more of the couch 216, image acquisition device 320, and radiotherapy device 330. The control circuitry of the radiation delivery device 330 may be integrated within the system 300 or remote from it.

During a radiotherapy treatment session, a patient may be positioned on couch 216. System 300 may then move couch 216 into the treatment area defined by the magnet 321, coils 325, 326, and chassis 335. Control circuitry may then control radiation source 331, MLC 332, and the chassis motor(s) to deliver radiation to the patient through the window between coils 325 and 326 according to a radiotherapy treatment plan.

**FIG. 2A, FIG. 2B,** and **FIG. 3** generally illustrate examples of a radiation therapy device configured to provide radiotherapy treatment to a patient, using a configuration where a radiation therapy output can be rotated around a central axis (e.g., an axis "*A*"). Other radiation therapy output configurations can be used. For example, a radiation therapy output can be mounted to a robotic arm or manipulator having multiple degrees of freedom. In yet another example, the therapy output can be fixed, such as located in a region laterally separated from the patient, and a platform supporting the patient can be used to align a radiation therapy isocenter with a specified target locus within the patient.

**FIG. 4** illustrates a contrasting example of a Leksell Gamma Knife radiotherapy device 430, which provides such radiotherapy treatment by means of gamma radiation. As a brief overview of a Gamma Knife device, radiation is emitted from a large number of fixed radioactive sources and is focused by means of collimators, i.e. passages or channels for obtaining a beam of limited cross section, towards a defined target or treatment volume. Each of the sources provides a dose of gamma radiation which is insufficient to damage intervening tissue. However, tissue destruction occurs where the radiation beams from all or some radiation sources intersect or converge, causing the radiation to reach tissue-destructive levels. The point of convergence is hereinafter referred to as the "isocenter" but may also be referred to as a "focus point".

As shown in FIG. 4, in a radiotherapy treatment session, a patient 402 may wear a coordinate frame 420 to fixate the patient's body part (e.g., the head) undergoing surgery or radiotherapy. Coordinate frame 420 and a patient positioning system 422 may establish a spatial coordinate system, which may be used while imaging a patient or during radiation surgery. Radiotherapy device 430 may include a protective housing 414 to enclose a plurality of radiation sources 412. Radiation sources 412 may generate a plurality of radiation beams (e.g., beamlets) through beam channels 416. The plurality of radiation beams may be configured to focus on an isocenter 310 from different directions. While each individual radiation beam may have a relatively low intensity, isocenter 310 may receive a relatively high level of radiation when multiple doses from different radiation beams accumulate at isocenter 310. In certain examples, isocenter 310 may correspond to a target under surgery or treatment, such as a tumor.

Other types of radiotherapy devices (not illustrated) use protons and/or ions to deliver the radiotherapy treatment. The direction and shape of the radiation beam should be accurately controlled to ensure that the tumor receives the prescribed radiation dose, and the radiation from the beam should minimize damage to the surrounding healthy tissue, especially the organ(s) at risk (OARs). Thus, treatment planning is used to design and control radiation beam parameters, and a radiotherapy device effectuates a treatment by delivering a spatially varying dose distribution to the patient.

Treatment plan optimization for radiation therapy, such as for Gamma knife radiosurgery, aims at maximizing the dose delivered to the target volume within the patient (e.g. in treatment of tumors) at the same time as the dose delivered to adjacent normal tissues is minimized. In treatment plan optimization, the delivered radiation dose is mainly limited by two competing factors: the first one is delivering a high dose to the target volume and the second one is delivering low dose to the surrounding normal tissues. The treatment plan optimization is a process including optimizing the number of shots being used, the sector-collimator combinations, the shot times, and the position of the shot (i.e. the compact dose distribution at the isocenter). Clearly, the irregularity and size of a target volume greatly influence the number of shots needed and the size of the shots being used to optimize the treatment. Thus, for gamma knife radiotherapy, the selected isocenter locations and their corresponding shots for a given case constitutes a treatment plan.

As noted above, a dose optimization process for the development of a radiotherapy treatment plan can involve two passes. For instance, two passes are applied in treatment planning software (e.g., the ELEKTA Leksell Gamma Knife® Lightning treatment planning platform) when optimizing treatment plans for a gamma knife treatment.

In the first pass, the optimal dose distribution corresponding to a set of weights is calculated based on dose points (target, ring, low-dose, and OAR) sampled from volumes determined geometrically based on the corresponding binary volumes. The purpose of the low-dose points is to limit the volume of the regions receiving more than some fraction of the prescription dose (e.g., more than half of the prescription dose, $V_{D_P/2}$). However, since the geometric description of the low-dose volume is not sufficiently accurate to encompass the relevant region (i.e., around where the optimized dose falls to below half the prescription dose) for all targets and weight combinations, a second pass is necessary.

In the second pass, the low-dose points are recalculated by sampling from the volume receiving between $\eta_l$ and $\eta_u$ times the prescription dose in the first pass optimization. (In practice, this volume is often divided into two or more separate volumes from which two or more separate sets of low-dose points are sampled. However, for simplicity, the following techniques are described with reference to a single low-dose volume and one set of low-dose points.) The remaining dose points are sampled from the same volumes as in the first pass.

With conventional approaches for radiotherapy planning, the planner would control trade-offs by selecting importance weights for the different metrics. Although this generally reduces planning time compared to manual planning, it is an iterative process; metrics produced for a set of weights differ for each patient case (due to anatomical differences), and the planner would likely need to tweak the weights and re-run the optimization several times to achieve a plan with desired trade-offs. To speed up planning and ensure plan quality is not compromised by the planner overlooking a 'better' set of weights due to time constraints, it is desirable to create plans corresponding to many sets of weights simultaneously (i.e., generating many points on the Pareto surface) as described above. The planner would then 'navigate' the corresponding set of plans in real time and choose the most desirable one.

The following approaches specifically enable the use of ADMM or similar methods to optimize, efficiently and in parallel, multiple radiotherapy treatment plans in minimal time (e.g., a few seconds). In an example, ADMM (an acronym for alternating direction method of multipliers) is applied to parallelize execution of linear or quadradic programming equations. ADMM may follow the algorithmic approach discussed in Boyd et al., Distributed Optimization and Statistical Learning via the Alternating Direction Method of Multipliers, Now Publishers Inc. (2011), and applied as a batch LP solver in Nair et al., Solving Mixed Integer Programs Using Neural Networks, arXiv:2012.13349 (2020).

The ADMM algorithm solves convex or non-convex optimization problems by breaking them into smaller pieces, each of which is then easier to handle. The ADMM is well suited for use as a batch LP solver. In an example, the optimization problem can be expressed as:

$$\begin{aligned} &\textbf{minimize} \quad f(x) + g(z) \\ &\textbf{subject to } x = z \end{aligned}$$

**(EQUATION 3)**

With this optimization problem in mind, the ADMM solver can be expressed as:

for any $\rho > 0$:

$$\begin{aligned} x^{k+1} &= \arg\min_x \left( f(x) + (\rho/2)\|x - z^k - \lambda^k\|_2^2 \right) \\ z^{k+1} &= \arg\min_z \left( g(z) + (\rho/2)\|x^{k+1} - z - \lambda^k\|_2^2 \right) \\ \lambda^{k+1} &= \lambda^k + x^{k+1} - z^{k+1}. \end{aligned}$$

**(EQUATION 4)**

For a LP problem this can be re-formulated as

$$x^{k+1} = K(z^k + \lambda^k) + \Gamma_\rho$$

$$z^{k+1} = \Pi_w(x^{k+1} - \lambda^k)$$

$$\lambda^{k+1} = \lambda^k - (x^{k+1} - z^{k+1})$$

**(EQUATION 5)**

With this formulation,

$$Q = \begin{pmatrix} 1 & A^T \\ A & -1 \end{pmatrix}^{-1}, \mathrm{P} = \begin{pmatrix} 0 & 0 \\ 0 & 1 \end{pmatrix} \quad \mathrm{K} = \mathrm{Q}+\mathrm{P};$$

$$\Gamma_\rho = Q \begin{pmatrix} -c_\rho \\ 0 \end{pmatrix}, c_\rho = \frac{c}{\rho}$$

**(EQUATION 6)**

Where A is the constraint matrix and c is the linear cost function vector. $\Pi_w$ is an operator that projects points onto the feasible set and is dependent on the weights multiplying the objective function terms in the objection function. It projects each element in a vector to a closed or half-open interval. Thus, each optimization is carried out with its unique projection operator which is quickly calculated. The operator K is, however, common for all optimizations but more time demanding to calculate.

In an example, the use of the ADMM algorithm to solve a radiotherapy problem, may be summarized with the following operations:

1) Calculate the K-operator and represent it, for example, explicitly or in factorized form.
2) Transfer the K-operator to each node.
3) Run the iteration scheme, such as described with reference to Equation 5, until the difference in variables between two successive iterations is less than a pre-defined threshold. In an example, the iteration scheme involves matrix multiplications (for a problem matrix) and a projection.

The ADMM formulation can provide an equally accurate solution as other solution methods but may require longer time for processing if particularly accurate solutions are desirable. However, it will be understood that ADMM provides a reasonably exact solution after a few iterations, and may be further used for Pareto navigation. Such a chosen plan can then be improved upon by other optimization methods (Simplex, interior point, ADMM,...).

Although multiple radiotherapy plans can be optimized in parallel using ADMM, a conventional use of a two-pass optimization prevents use of ADMM on the second pass because the problem matrix of the second pass differs for different sets of weights (since each set of weights gives rise to a different estimate of the low-dose region after the first pass). In addition, the solutions of a conventional two-pass optimization are not guaranteed to form a convex set with respect to the objective function terms, since a different problem is solved for each set. However, the following provides various adaptations that enable use of ADMM even with a two-pass optimization procedure.

**FIG. 5** provides a high-level view of radiotherapy treatment planning workflow operations. Specifically, this workflow uses parallel processing for radiotherapy problem optimization, that can generate sufficiently many Pareto-optimal solutions for a radiotherapy problem.

The operations in FIG. 5, in more detail, illustrate how radiotherapy problem information 510 for a human subject are provided with the definition of information such as target regions of treatment 512 and organ at risk regions 514. Other information relevant to the radiotherapy problem may include radiotherapy machine information 520 such as machine capabilities 522.

A suitable dose distribution to be delivered with radiotherapy problem is then optimized and solved with the depicted radiotherapy problem optimization 530. Such optimization and solution may occur with the use of parallel processing hardware (e.g., a first configuration of parallel processing hardware 535A and a second configuration of the parallel processing hardware 535B), and the transformation or modification of the radiotherapy problems for use with the parallel processing hardware.

As noted above, the parallel processing hardware may include a set of one or more graphics processing units (GPUs) or other processor units, and the relevant transformation of the problem may include the use of an alternating direction method of multipliers technique to solve linear programming equations. The use of ADMM may transform the linear programming equations into matrix and projection operations. In a first pass 531, the optimization 530 may generate multiple radiotherapy treatment solutions (optimized with respect to their corresponding sets of weights and the initial geometrical approximation of the low-dose region) with use of ADMM operations, in parallel, on the first configuration of parallel processing hardware 535A; then, in a second pass 532, the optimization 530 may generate new sets of radiotherapy treatment solutions (optimized with respect to their corresponding sets of weights and the more accurate approximation of the low-dose region from the first pass) with use of ADMM operations, in parallel, on the second configuration of parallel processing hardware 535B.

The optimization 530 produces a plurality of optimized (e.g., Pareto-optimal) radiotherapy solutions 540, which may consist of a Pareto surface or frontier of all (or approximately all) available optimized solutions. One or more of these solutions may be selected and refined with operation 550, and used for generation of a particular radiotherapy treatment plan with operation 560. The generation of the radiotherapy treatment plan with operation 560 (and, related selection or modification of the radiotherapy solution) may be dependent on machine capabilities 522. Finally, radiotherapy treatment may be delivered on a radiotherapy modality using the generated treatment plan.

As noted above, the use of ADMM transforms linear programming equations into matrix and projection operations (e.g., with a problem matrix) which can be evaluated by parallel processing hardware. One way of ensuring the same problem matrix for a problem with m distinct sets of weights, $w_i$, would be to take all the low-dose points, $p_i^{\mathrm{ld}}$, and incorporating their union, $P^{\mathrm{ld}} = \cup_i p_i^{\mathrm{ld}}$, in the matrix. For each $w_i$, the low-dose points belonging to all other sets of weights could then be 'switched off' without affecting the problem matrix. The switching off is achieved by setting the upper bounds (corresponding to the weights in the primal problem) of the variables of the dual problem corresponding to the undesired dose points to zero. Unless care is taken, however, this would make the problem infeasibly large. However, the overlap between low-dose volumes, $V_i$, corresponding to different sets of weights is typically substantial.

In many scenarios, the union of all low-dose volumes typically is about twice that of the maximum low-dose volume for any single set of weights: $|\cup_i \mathcal{V}_i| \cong 2 \max_i |\mathcal{V}_i|$. Assuming all low-dose volumes are sampled with the same sampling density, $\rho$, one approach may involve sampling $\rho|\cup_i V_i|$ points from inside $\cup_i V_i$ and include all these in the problem matrix. For problem *i*, the value of the upper bound for a point found inside $V_i$ is set to $w_i^{\mathrm{ld}}/N_i$, where and $N_i$ is the number of sampled low-dose points inside $V_i$. The upper bound for the remaining low-dose points are then set to zero. Assuming the union of low-dose volumes is about twice the size of any individual low-dose volume, this would double the number of low-dose points rather than increasing them by a factor of *m*.

A further optimization for keeping the results of the second pass convex with respect to the weights, while producing suitable results, is as follows. The effectiveness of this optimization depends roughly on how much the volume receiving, for example half the prescription dose, $V_{D_P/2}$, changes shape in the second pass. In this optimization, let $V_i$ be the low-dose volume for weight set $w_i$ obtained after the first pass and let $\hat{V}_i$ be the corresponding volume after the second pass. Assume that the difference between $V_i$ and $\hat{V}_i$ is small (i.e., repeated selection of a new low-dose region based on the previous optimization converges quickly). The magnitude of the contribution to the change in the low-dose term of the objective function from the *j*:th low-dose point in $p_i^{\mathrm{ld}}$, $p_{i,j}^{\mathrm{ld}}$, can then be approximated by $\delta_{i,j}|\nabla D_{i,j}|$, where $\delta_{i,j}$ is the local shift of the dose distribution perpendicular to the isodose line and $\nabla D_{i,j}$ is the dose gradient at the point. Thus, the magnitude of the change in the objective function from an isotropic outward shift in the $\eta_l D_P$ isodose surface by a distance $\delta$ becomes $(w_i^{\mathrm{ld}}/N_i^{\mathrm{ld}})\delta \sum_j |\nabla D_{i,j}|$, where the sum is taken over all, $N_i^{\mathrm{ld}}$, points in $p_i^{\mathrm{ld}}$ and any additional normalizations have been left out. The corresponding magnitude of the change in objective function if the union of low-dose points, $P^{\mathrm{ld}}$, had been used would instead be $(w_i^{\mathrm{ld}}/N_{\mathrm{U}}^{\mathrm{ld}})\delta \sum_{\hat{p}_j} |\nabla D_{\hat{p}_j}|$, with $N_{\mathrm{U}}^{\mathrm{ld}}$ the number of points in $P^{\mathrm{ld}}$ and the sum this time taken over all points $\hat{p} = (p \in P^{\mathrm{ld}} | D(p) \geq \eta_l D_P)$ where $\eta_l$ is the low-dose threshold.

Under a further assumption that the relative 'thicknesses' of the shells of low-dose points in $p_i^{\mathrm{ld}}$ and $P^{\mathrm{ld}}$ are roughly equal in all directions from the target, the behavior of the low-dose objective term is matched by including all $P^{\mathrm{ld}}$ with that including only $p_i^{\mathrm{ld}}$ in the vicinity of the expected solution. To achieve this, substitute $\tilde{w}_i^{\mathrm{ld}}$ for $w_i^{\mathrm{ld}}$ in the low-dose term when using all $P^{\mathrm{ld}}$, where

$$\tilde{w}_i^{\mathrm{ld}} = w_i^{\mathrm{ld}} \frac{N_{\mathrm{U}}^{\mathrm{ld}} \sum_j |\nabla D_{i,j}|}{N_i^{\mathrm{ld}} \sum_{\hat{p}_j} |\nabla D_{\hat{p}_j}|}$$

**(EQUATION 7)**

Doing so for all *i* allows use of the same set of low-dose points, $P^{\mathrm{ld}}$, for all sets of weights in the second pass, and for each low-dose point to have the same weight for a given *i*. This should ensure convexity of the solution with respect to the low-dose term.

**FIG. 6** illustrates an example workflow for performing multi-pass operations of radiology problem optimization with parallel processing, where multiple passes are employed to improve the estimate of the low-dose region. This workflow includes the use of a first pass 610 to produce approximate solutions for the optimization problem corresponding to different sets of weights. From the approximate solutions, new low-dose regions are obtained, which are used in the second pass 620 to produce new solutions which better control the spillage dose to regions of healthy tissue away from the target.

In the following example, the problem matrix is computed (based on the constraint matrix A in Equation 6), and then the optimization problem is solved by repeated multiplication with this matrix and projection back onto the feasible set. Because the weights of the primal problem enters the dual problem as upper bounds, these weights only affect the projection step but not the problem matrix. Hence, for each pass, the same problem matrix can be used in the multiplication for all sets of weights, and then only change the projection step for each set of weights, to enable the use of parallel optimization with ADMM.

In the first pass 610, at operation 611, a first problem matrix is constructed based on dose points selected from the target, ring, geometric estimate of the low-dose region, and OARs, as previously described. At operation 612, optimization with respect to multiple weight sets is performed, by solving the first set of problems represented in the first problem matrix on parallel processing hardware using ADMM (e.g., with parallel optimization). Based on the solutions from this first pass 610, the low-dose regions corresponding to different weight sets are then combined (e.g., with a union operation) at operation 615 to create a refined, common low-dose region.

In the second pass 620, at operation 621, a second problem matrix is constructed in the same way as before, but this time with low-dose points selected from the refined low-dose region obtained after the first optimization pass. Then, at operation 622, optimization may be performed in parallel by solving the second set of problems represented in the second problem matrix. This may include a parallel optimization 623 which, for each weight set, involves activating only low-dose points from within the low-dose region corresponding to that weight set; or a parallel optimization 624 in which all low-dose points are active but a new low-dose weight is calculated for each weight set. With parallel optimization 623, all low-dose points are provided in the problem matrix, but only a subset are "switched on" (with a non-zero upper bound), corresponding to the individual low-dose region for each set of weights (i.e., the upper bound is set to zero for those that are "switched off"). Alternatively, with the parallel optimization 624, the union of all low-dose points are also provided in the problem matrix but a new low-dose weight is calculated for each weight set according to EQUATION 7. This new low-dose weight is applied to all low-dose points in the union, and is calculated to mimic behavior of the original radiotherapy problem.

**FIG. 7A** illustrates example results from serial processing of radiology problem optimization. As shown, a target volume for radiotherapy treatment is represented by regions 721, 722, 723. The target volume is surrounded by a low-dose volume, represented by regions 711, 712, 713. The low-dose volume includes multiple low-dose points (illustrated as dots in regions 711, 712, 713) which are sampled during the first optimization pass. Before optimization, all of the volumes are the same for the different weights.

After the first optimization pass, the target volume is represented by regions 741, 742, 743, and the low-dose volume is represented by regions 731, 732, 733, for the respective weight sets. As will be understood, since the size of the low-dose volume differs, a common problem matrix cannot be constructed for the second optimization pass; and thus, serial processing is required (i.e., parallel processing cannot be performed).

**FIG. 7B** illustrates example results from parallel processing of radiology problem optimization. Here, the target volume for radiotherapy treatment is represented by region 761, and a corresponding low-dose volume represented by region 751. Since all weight sets share the same low-dose approximation, the same low-dose points are used for all weight sets in the first pass. This first pass occurs with the use of parallel optimization operations 770.

Next, FIG. 7B illustrates the results of the first pass of the parallel optimization operations 770. This includes the target volume (with the same size) represented by regions 762, 763, 764, and the low-dose volume (with different sizes) represented by regions 752, 753, 754. In an example, these low-dose regions are combined (e.g., unioned) and sampled, with combination operations 780. (In this context, being sampled means that a number of dose points are selected from within this union of low-dose volumes). This produces a representation of the target volume (remaining the same size) with region 765, and a representation of the refined low-dose volume with region 745. After being combined and sampled, a second pass can occur with the use of parallel optimization operations 790.

In further examples, specific types of optimization may be performed in the second pass. The optimization in the second pass may include operation 791 for activating dose points corresponding to a particular weight set (corresponding to operation 623, above); or an operation 792 which calculates new low dose weights for each weight set (corresponding to operation 624, above).

**FIG. 8** illustrates a flowchart 800 of a method of radiotherapy treatment planning based on the techniques discussed above. For instance, the following features of flowchart 800 may be integrated or adapted with the optimization operations discussed with reference to FIG. 5, sequential operations discussed with reference to FIG. 6, to produce optimized outcomes such as discussed with reference to FIG. 7B.

Operation 810 begins with operations to obtain a set of radiotherapy problems, with the radiotherapy problems defining various parameters for delivery of a radiotherapy treatment from a radiotherapy machine. Such radiotherapy problems may be adjustable via parameters, and may be optionally received with a request to solve the radiotherapy problems (a set of first optimization problems). Such parameters considered by the problems may define constraints for at least one target area and at least one low-dose area in the at least one treatment area. Such parameters may also relate to radiation delivery parameters of a radiotherapy treatment machine.

Operation 820 proceeds with operations to perform a dose optimization for the obtained radiotherapy problem. This dose optimization may include:

At operation 822, converting the set of first optimization problems into a first problem matrix.

At operation 824, performing a first pass of the dose optimization on the first problem matrix, such as by solving the first optimization problems represented in the first problem matrix in parallel on parallel processing hardware. In this operation, the first pass will produce a first set of multiple solutions, corresponding to a first plurality of multiple sets of weights, to the first optimization problems.

At operation 826, combining the first set of multiple solutions to the first optimization problems to produce a set of second optimization problems for providing the radiotherapy treatment. The set of the second optimization problems is likewise defined by a second plurality of parameters. As noted above, combining may include combining low-dose regions from the first set of multiple solutions into a refined (common) region from which low-dose points for the second optimization problems are selected.

At operation 828, converting the second optimization problems into a second problem matrix.

At operation 830, performing a second pass of dose optimization by solving the second optimization problems represented in the second problem matrix in parallel on the parallel processing hardware. Here, the second pass is to produce a second set of multiple solutions, corresponding to a second plurality of multiple sets of weights, to the second optimization problems.

As explained in the examples above, the multiple sets of weights, corresponding to the multiple solutions from the first pass or second pass, may relate to points defined for at least one low-dose volume. For instance, the dose to be delivered in the low-dose region may be a fraction of a dose to be delivered in a target region.

At operation 840, one or more dose-optimized radiotherapy solutions are produced, from the two-pass operations (822, 824, 826, 828, 830). This solution (or one of the set of solutions) may be identified as the optimal or "best" solution for use in radiotherapy treatment. At operation 850, one of the plurality of solutions is further evaluated, selected, and potentially improved (e.g., further optimized). Finally, at operation 860, a treatment plan to control the radiotherapy machine is generated based on the optimized radiotherapy solution.

Although not depicted in the drawings, various forms of user interfaces or representations may be provided to enable a representation of a solution space to the radiotherapy problem, including different areas that are optimized. This may be provided in a graphical user interface having functionality to configure the treatment plan, and to receive and output data related to the treatment plan. User interaction may be obtained in such a user interface for modifying the particular set of parameters. As will be understood, a variety of interactive navigation functions may be provided, such as an approximation of the Pareto surface that lets a user explore estimates of solutions corresponding to new or different parameter values. Further techniques for navigation of a Pareto surface are discussed in Patent Application No. PCT/EP2022/053440, publication WO 2023/151817 A1, titled EXPLORATION OF PARETO-OPTIMAL RADIOTHERAPY PLANS, filed February 11, 2022.

**FIG. 9** illustrates a block diagram of an example of a machine 900 on which one or more of the methods as discussed herein can be implemented. In one or more examples, one or more items of the radiotherapy processing computing system 110 can be implemented by the machine 900. In alternative examples, the machine 900 operates as a tandalone device or may be connected (e.g., networked) to other machines. In one or more examples, the radiotherapy processing computing system 110 can include one or more of the items of the machine 900. In a networked deployment, the machine 900 may operate in the capacity of a server or a client machine in server-client network environment, or as a peer machine in a peer-to-peer (or distributed) network environment. The machine may be a personal computer (PC), server, a tablet, smartphone, a web appliance, edge computing device, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein.

The example machine 900 includes processing circuitry or processor 902 (e.g., a CPU, a graphics processing unit (GPU), an ASIC, circuitry, such as one or more transistors, resistors, capacitors, inductors, diodes, logic gates, multiplexers, buffers, modulators, demodulators, radios (e.g., transmit or receive radios or transceivers), sensors 921 (e.g., a transducer that converts one form of energy (e.g., light, heat, electrical, mechanical, or other energy) to another form of energy), or the like, or a combination thereof), a main memory 904 and a static memory 906, which communicate with each other via a bus 908. The machine 900 (e.g., computer system) may further include a video display device 910 (e.g., a liquid crystal display (LCD) or a cathode ray tube (CRT)). The machine 900 also includes an alphanumeric input device 912 (e.g., a keyboard), a user interface (UI) navigation device 914 (e.g., a mouse), a disk drive or mass storage unit 916, a signal generation device 918 (e.g., a speaker), and a network interface device 920.

The disk drive unit 916 includes a machine-readable medium 922 on which is stored one or more sets of instructions and data structures (e.g., software) 924 embodying or utilized by any one or more of the methodologies or functions described herein. The instructions 924 may also reside, completely or at least partially, within the main memory 904 and/or within the processor 902 during execution thereof by the machine 900, the main memory 904 and the processor 902 also constituting machine-readable media.

The machine 900 as illustrated includes an output controller 928. The output controller 928 manages data flow to/from the machine 900. The output controller 928 is sometimes called a device controller, with software that directly interacts with the output controller 928 being called a device driver.

While the machine-readable medium 922 is shown in an example to be a single medium, the term "machine-readable medium" may include a single medium or multiple media (e.g., a centralized or distributed database, and/or associated caches and servers) that store the one or more instructions or data structures. The term "machine-readable medium" shall also be taken to include any tangible medium that is capable of storing, encoding or carrying instructions for execution by the machine and that cause the machine to perform any one or more of the methodologies of the present disclosure, or that is capable of storing, encoding or carrying data structures utilized by or associated with such instructions. The term "machine-readable medium" shall accordingly be taken to include, but not be limited to, solid-state memories, and optical and magnetic media. Specific examples of machine-readable media include non-volatile memory, including by way of example semiconductor memory devices, e.g., Erasable Programmable Read-Only Memory (EPROM), EEPROM, and flash memory devices; magnetic disks such as internal hard disks and removable disks; magneto-optical disks; and CD-ROM and DVD-ROM disks.

The instructions 924 may further be transmitted or received over a communications network 926 using a transmission medium. The instructions 924 may be transmitted using the network interface device 920 and any one of a number of well-known transfer protocols (e.g., HTTP). Examples of communication networks include a LAN, a WAN, the Internet, mobile telephone networks, Plain Old Telephone (POTS) networks, and wireless data networks (e.g., Wi-Fi and 4G/5G data networks). The term "transmission medium" shall be taken to include any intangible medium that is capable of storing, encoding or carrying instructions for execution by the machine, and includes digital or analog communications signals or other intangible media to facilitate communication of such software.

As used herein, "communicatively coupled between" means that the entities on either of the coupling must communicate through an item therebetween and that those entities cannot communicate with each other without communicating through the item.

## Additional Notes

The above detailed description includes references to the accompanying drawings, which form a part of the detailed description. The drawings show, by way of illustration but not by way of limitation, specific embodiments in which the disclosure can be practiced.

In this document, the terms "a," "an," "the," and "said" are used when introducing elements of aspects of the disclosure or in the embodiments thereof, as is common in patent documents, to include one or more than one or more of the elements, independent of any other instances or usages of "at least one" or "one or more." In this document, the term "or" is used to refer to a nonexclusive or, such that "A or B" includes "A but not B," "B but not A," and "A and B," unless otherwise indicated.

In the appended claims, the terms "including" and "in which" are used as the plain-English equivalents of the respective terms "comprising" and "wherein." Also, in the following claims, the terms "comprising," "including," and "having" are intended to be open-ended to mean that there may be additional elements other than the listed elements. Moreover, in the following claims, the terms "first," "second," and "third," and so forth, are used merely as labels, and are not intended to impose numerical requirements on their objects.

Embodiments of the disclosure may be implemented with computer-executable instructions. The computer-executable instructions (e.g., software code) may be organized into one or more computer-executable components or modules. Aspects of the disclosure may be implemented with any number and organization of such components or modules.

Method examples (e.g., operations and functions) described herein can be machine or computer-implemented at least in part (e.g., implemented as software code or instructions). Some examples can include a computer-readable medium or machine-readable medium encoded with instructions operable to configure an electronic device to perform methods as described in the above examples. An implementation of such methods can include software code, such as microcode, assembly language code, a higher-level language code, or the like (e.g., "source code"). Such software code can include computer-readable instructions for performing various methods (e.g., "object" or "executable code"). The software code may form portions of computer program products. Software implementations of the embodiments described herein may be provided via an article of manufacture with the code or instructions stored thereon, or via a method of operating a communication interface to send data via a communication interface (e.g., wirelessly, over the internet, via satellite communications, and the like).

Further, the software code may be tangibly stored on one or more volatile or non-volatile computer-readable storage media during execution or at other times. These computer-readable storage media may include any mechanism that stores information in a form accessible by a machine (e.g., computing device, electronic system, and the like), such as, but are not limited to, floppy disks, hard disks, removable magnetic disks, any form of magnetic disk storage media, CD-ROMS, magnetic-optical disks, removable optical disks (e.g., compact disks and digital video disks), flash memory devices, magnetic cassettes, memory cards or sticks (e.g., secure digital cards), RAMs (e.g., CMOS RAM and the like), recordable/non-recordable media (e.g., read only memories (ROMs)), EPROMS, EEPROMS, or any type of media suitable for storing electronic instructions, and the like. Such computer-readable storage medium is coupled to a computer system bus to be accessible by the processor and other parts of the OIS.

In an embodiment, the computer-readable storage medium may have encoded a data structure for treatment planning, wherein the treatment plan may be adaptive. The data structure for the computer-readable storage medium may be at least one of a Digital Imaging and Communications in Medicine (DICOM) format, an extended DICOM format, an XML format, and the like. DICOM is an international communications standard that defines the format used to transfer medical image-related data between various types of medical equipment. DICOM RT refers to the communication standards that are specific to radiation therapy.

In various embodiments of the disclosure, the method of creating a component or module can be implemented in software, hardware, or a combination thereof. The methods provided by various embodiments of the present disclosure, for example, can be implemented in software by using standard programming languages such as, for example, C, C++, C#, Java, Python, CUDA programming, and the like; and combinations thereof. As used herein, the terms "software" and "firmware" are interchangeable, and include any computer program stored in memory for execution by a computer.

A communication interface includes any mechanism that interfaces to any of a hardwired, wireless, optical, and the like, medium to communicate to another device, such as a memory bus interface, a processor bus interface, an Internet connection, a disk controller, and the like. The communication interface can be configured by providing configuration parameters and/ or sending signals to prepare the communication interface to provide a data signal describing the software content. The communication interface can be accessed via one or more commands or signals sent to the communication interface.

The present disclosure also relates to a system for performing the operations herein. This system may be specially constructed for the required purposes, or it may comprise a general purpose computer selectively activated or reconfigured by a computer program stored in the computer. The order of execution or performance of the operations in embodiments of the disclosure illustrated and described herein is not essential, unless otherwise specified. That is, the operations may be performed in any order, unless otherwise specified, and embodiments of the disclosure may include additional or fewer operations than those disclosed herein. For example, it is contemplated that executing or performing a particular operation before, contemporaneously with, or after another operation is within the scope of aspects of the disclosure.

In view of the above, it will be seen that the several objects of the disclosure are achieved and other advantageous results attained. Having described aspects of the disclosure in detail, it will be apparent that modifications and variations are possible without departing from the scope of aspects of the disclosure as defined in the appended claims. As various changes could be made in the above constructions, products, and methods without departing from the scope of aspects of the disclosure, it is intended that all matter contained in the above description and shown in the accompanying drawings shall be interpreted as illustrative and not in a limiting sense.

The above description is intended to be illustrative, and not restrictive. For example, the above-described examples (or one or more aspects thereof) may be used in combination with each other. In addition, many modifications may be made to adapt a particular situation or material to the teachings of the disclosure without departing from its scope. While the dimensions, types of materials and coatings described herein are intended to define the parameters of the disclosure, they are by no means limiting and are exemplary embodiments. Many other embodiments will be apparent to those of skill in the art upon reviewing the above description. The scope of the disclosure should, therefore, be determined with reference to the appended claims, which define the invention.

## Claims

**1.** A computer-implemented method for radiotherapy treatment planning, comprising:

obtaining a set of first optimization problems for providing radiotherapy treatment to a human subject, the first optimization problems defined by a first plurality of parameters;

performing dose optimization for delivery of the radiotherapy treatment to at least one treatment area of the human subject, the dose optimization comprising:

converting the first optimization problems into a first problem matrix;
performing a first pass of the dose optimization by solving, using a linear programming solver operating based on an alternating direction method of multipliers technique, the first optimization problems represented in the first problem matrix in parallel on parallel processing hardware, the first pass to produce a first set of multiple solutions, corresponding to a first plurality of multiple sets of weights, to the first optimization problems;
combining the first set of multiple solutions to the first optimization problems to produce a set of second optimization problems for providing the radiotherapy treatment, the second optimization problems defined by a second plurality of parameters;
converting the second optimization problems into a second problem matrix; and
performing a second pass of the dose optimization by solving, using the linear programming solver operating based on the alternating direction method of multipliers technique, the second optimization problems represented in the second problem matrix in parallel on the parallel processing hardware, the second pass to produce a second set of multiple solutions, corresponding to a second plurality of multiple sets of weights, to the second optimization problems; and

generating treatment plan data based on at least one solution of the second set of multiple solutions to the second optimization problems, wherein the treatment plan data is usable to control delivery of radiotherapy from a radiotherapy machine;
wherein the at least one treatment area includes a low-dose region and a target region, wherein a dose to be delivered in the low-dose region is a fraction of a dose to be delivered in the target region, and wherein combining the first set of multiple solutions to produce the second optimization problems comprises:
performing a union of the first set of multiple solutions to the first optimization problems for the low-dose region.

2. The method of claim 1, wherein the first and the second plurality of parameters define constraints for at least one target area and at least one low-dose area in the at least one treatment area.

3. The method of any of claims 1 to 2, wherein the first and second plurality of multiple sets of weights, corresponding to the first and second set of multiple solutions, relate to points defined for at least one low-dose volume.

4. The method of any of claims 1 to 3, wherein the first plurality of parameters and the second plurality of parameters relate to radiation delivery parameters of a radiotherapy treatment machine.

5. The method of any of claims 1 to 4, wherein solving the first optimization problems or the second optimization problems in parallel on the parallel processing hardware comprises, for a respective set of problems:

identifying parameterized linear programming equations from the respective set of problems; and
converting the parameterized linear programming equations for execution by the parallel processing hardware; and
wherein solving the respective set of problems in parallel comprises solving a plurality of the converted parameterized linear programming equations in parallel on the parallel processing hardware, to produce a plurality of solutions to the respective set of problems.

6. The method of claim 5, wherein converting the parameterized linear programming equations comprises applying the alternating direction method of multipliers technique, and wherein the alternating direction method of multipliers technique comprises transforming the converted parameterized linear programming equations to matrix and projection operations.

7. The method of any of claims 1 to 6, wherein the parallel processing hardware comprises a set of one or more graphics processing units (GPUs).

8. The method of any of claim 1 to 7, wherein each low-dose point selected from a common low-dose region of the first set of multiple solutions of the first optimization problems is represented in the second problem matrix, and wherein performing the second pass of the dose optimization includes assigning a non-zero upper bound to a solution vector in a subset of points corresponding a respective low-dose region for each set of weights.

9. The method of any of claims 1 to 7, wherein each low-dose point selected from a common low-dose region of the first set of multiple solutions of the first optimization problem is represented in the second problem matrix, and wherein performing the second pass of the dose optimization includes applying a new low-dose weight to all low-dose points in the union of the first set of multiple solutions of the first optimization problems.

**10.** The method of any of claims 1 to 7, wherein combining the first set of multiple solutions to produce the second optimization problems comprises:
performing a sampling of the union of the first set of multiple solutions to identify weights of the second plurality of parameters for the low-dose region.

**11.** The method of any of claims 1 to 10, further comprising:

selecting a solution to the second optimization problems based on an evaluation of the second set of multiple solutions, wherein the treatment plan data is generated based on the selected solution to the second optimization problems;
and optionally, wherein the selected solution to the second optimization problems provides an approximate solution, with the method further comprising receiving an additional optimization to the selected solution, wherein the treatment plan data is generated based on the additional optimization to the selected solution.

**12.** The method of any of claims 1 to 11, wherein the treatment plan data for the radiotherapy treatment comprises a set of treatment delivery parameters corresponding to capabilities of a radiotherapy treatment machine;

and optionally, wherein the radiotherapy treatment is to be provided with a Gamma knife, and the set of treatment delivery parameters comprises a set of isocenters used for delivery of the radiotherapy treatment;
and optionally, wherein the set of treatment delivery parameters further comprises timing for delivery of the radiotherapy treatment and a collimator sequence for the delivery of the radiotherapy treatment;
and optionally, wherein the radiotherapy treatment is provided with a Volumetric-modulated arc therapy (VMAT) or Intensity modulated radiation therapy (IMRT) using a Linac radiotherapy machine, and wherein the set of treatment delivery parameters comprises: a set of arc control points for one or more arcs, fluence fields, gantry speed, and dose rate along the one or more arcs.

**13.** A computer-readable storage medium comprising computer-readable instructions for radiotherapy treatment planning, wherein the instructions, when executed, cause a computing machine to perform any of the methods of claims 1 to 12.

**14.** A computing system configured for radiotherapy treatment planning, the system comprising:

one or more parallel processing hardware devices;
one or more memory devices to store data for providing radiotherapy treatment to a human subject; and
one or more processors configured to perform any of the methods of claims 1 to 12.

**Patentansprüche**

**1.** Computerimplementiertes Verfahren zur Strahlentherapiebehandlungsplanung, umfassend:

Erhalten eines Satzes von ersten Optimierungsproblemen zum Bereitstellen einer Strahlentherapiebehandlung für ein menschliches Subjekt, wobei die ersten Optimierungsprobleme durch eine erste Vielzahl von Parametern definiert sind;
Durchführen einer Dosisoptimierung zur Verabreichung der Strahlentherapiebehandlung an mindestens einen Behandlungsbereich des menschlichen Subjekts, wobei die Dosisoptimierung Folgendes umfasst:

Umwandeln der ersten Optimierungsprobleme in eine erste Problemmatrix;
Durchführen eines ersten Durchlaufs der Dosisoptimierung durch Lösen, unter Verwendung eines linearen Programmierlösers, der basierend auf einer Technik der wechselseitigen Richtungsmethode von Multiplikatoren arbeitet, der ersten Optimierungsprobleme, die in der ersten Problemmatrix dargestellt sind, in paralleler Weise auf Parallelverarbeitungshardware, wobei der erste Durchlauf dazu dient, einen ersten Satz von mehreren Lösungen, die einer ersten Vielzahl von mehreren Sätzen von Gewichtungen entsprechen, für die ersten Optimierungsprobleme zu erzeugen;
Kombinieren des ersten Satzes von mehreren Lösungen mit den ersten Optimierungsproblemen, um einen Satz von zweiten Optimierungsproblemen zum Bereitstellen der Strahlentherapiebehandlung zu erzeugen, wobei die zweiten Optimierungsprobleme durch eine zweite Vielzahl von Parametern definiert sind;
Umwandeln der zweiten Optimierungsprobleme in eine zweite Problemmatrix; und
Durchführen eines zweiten Durchlaufs der Dosisoptimierung durch Lösen, unter Verwendung des linearen Programmierlösers, der basierend auf der Technik der wechselseitigen Richtungsmethode von Multiplikatoren arbeitet, der zweiten Optimierungsprobleme, die in der zweiten Problemmatrix dargestellt sind, in paralleler Weise auf der Parallelverarbeitungshardware, wobei der zweite Durchlauf dazu dient, einen zweiten Satz von mehreren Lösungen, die einer zweiten Vielzahl von mehreren Sätzen von Gewichtungen entsprechen, für die zweiten Optimierungsprobleme zu erzeugen; und
Erzeugen von Behandlungsplandaten basierend auf mindestens einer Lösung des zweiten Satzes von mehreren Lösungen für die zweiten Optimierungsprobleme, wobei die Behandlungsplandaten verwendbar sind, um die Verabreichung einer Strahlentherapie von einer Strahlentherapiemaschine zu steuern;
wobei der mindestens eine Behandlungsbereich einen Niedrigdosisbereich und einen Zielbereich umfasst, wobei eine im Niedrigdosisbereich abzugebende Dosis ein Bruchteil einer im Zielbereich abzugebenden Dosis ist und wobei das Kombinieren des ersten Satzes von mehreren Lösungen zum Erzeugen der zweiten Optimierungsprobleme Folgendes umfasst:
Durchführen einer Vereinigung des ersten Satzes von mehreren Lösungen für die ersten Optimierungsprobleme für den Niedrigdosisbereich.

**2.** Verfahren nach Anspruch 1, wobei die erste und die zweite Vielzahl von Parametern Einschränkungen für mindestens einen Zielbereich und mindestens einen Niedrigdosisbereich in dem mindestens einen Behandlungsbereich definieren.

**3.** Verfahren nach einem der Ansprüche 1 bis 2, wobei sich die erste und die zweite Vielzahl von mehreren Mengen von Gewichtungen, die dem ersten und dem zweiten Satz von mehreren Lösungen entsprechen, auf Punkte beziehen, die für mindestens ein Niedrigdosisvolumen definiert sind.

**4.** Verfahren nach einem der Ansprüche 1 bis 3, wobei sich die erste Vielzahl von Parametern und die zweite Vielzahl von Parametern auf Strahlungsabgabeparameter einer Strahlentherapiebehandlungsmaschine beziehen.

**5.** Verfahren nach einem der Ansprüche 1 bis 4, wobei das Lösen der ersten Optimierungsprobleme oder der zweiten Optimierungsprobleme in paralleler Weise auf der Parallelverarbeitungshardware für einen jeweiligen Satz von Problemen Folgendes umfasst:

Identifizieren parametrisierter linearer Programmiergleichungen aus dem jeweiligen Satz von Problemen; und
Umwandeln der parametrisierten linearen Programmiergleichungen zur Ausführung durch die Parallelverarbeitungshardware; und
wobei das Lösen des jeweiligen Satzes von Problemen in paralleler Weise das Lösen einer Vielzahl der umgewandelten parametrisierten linearen Programmiergleichungen in paralleler Weise auf der Parallelverarbeitungshardware umfasst, um eine Vielzahl von Lösungen für den jeweiligen Satz von Problemen zu erzeugen.

**6.** Verfahren nach Anspruch 5, wobei das Umwandeln der parametrisierten linearen Programmiergleichungen das Anwenden der Technik der wechselseitigen Richtungsmethode von Multiplikatoren umfasst und wobei die Technik der wechselseitigen Richtungsmethode von Multiplikatoren das Transformieren der umgewandelten parametrisierten linearen Programmiergleichungen in Matrix- und Projektionsoperationen umfasst.

**7.** Verfahren nach einem der Ansprüche 1 bis 6, wobei die Parallelverarbeitungshardware einen Satz aus einer oder mehreren Grafikverarbeitungseinheiten (GPUs) umfasst.

**8.** Verfahren nach einem der Ansprüche 1 bis 7, wobei jeder Niedrigdosispunkt, der aus einem gemeinsamen Niedrigdosisbereich des ersten Satzes von mehreren Lösungen der ersten Optimierungsprobleme ausgewählt ist, in der zweiten Problemmatrix dargestellt ist, und wobei das Durchführen des zweiten Durchgangs der Dosisoptimierung das Zuweisen einer von Null verschiedenen oberen Grenze zu einem Lösungsvektor in einer Teilmenge von Punkten beinhaltet, die einem jeweiligen Niedrigdosisbereich für jeden Satz von Gewichtungen entsprechen.

**9.** Verfahren nach einem der Ansprüche 1 bis 7, wobei jeder Niedrigdosispunkt, der aus einem gemeinsamen Niedrigdosisbereich des ersten Satzes von mehreren Lösungen des ersten Optimierungsproblems ausgewählt ist, in der zweiten Problemmatrix dargestellt ist, und wobei das Durchführen des zweiten Durchgangs der Dosisoptimierung das Anwenden einer neuen Niedrigdosisgewichtung auf alle Niedrigdosispunkte in der Vereinigung des ersten Satzes von mehreren Lösungen der ersten Optimierungsprobleme beinhaltet.

**10.** Verfahren nach einem der Ansprüche 1 bis 7, wobei das Kombinieren des ersten Satzes von mehreren Lösungen zum Erzeugen der zweiten Optimierungsprobleme Folgendes umfasst:
Durchführen einer Stichprobennahme der Vereinigung des ersten Satzes von mehreren Lösungen, um Gewichtungen der zweiten Vielzahl von Parametern für den Niedrigdosisbereich zu identifizieren.

**11.** Verfahren nach einem der Ansprüche 1 bis 10, ferner umfassend:

Auswählen einer Lösung für die zweiten Optimierungsprobleme basierend auf einer Bewertung des zweiten Satzes von mehreren Lösungen, wobei die Behandlungsplandaten basierend auf der ausgewählten Lösung für die zweiten Optimierungsprobleme erzeugt werden;
und wobei optional die ausgewählte Lösung für die zweiten Optimierungsprobleme eine ungefähre Lösung bereitstellt, wobei das Verfahren ferner das Empfangen einer zusätzlichen Optimierung für die ausgewählte Lösung umfasst, wobei die Behandlungsplandaten basierend auf der zusätzlichen Optimierung für die ausgewählte Lösung erzeugt werden.

**12.** Verfahren nach einem der Ansprüche 1 bis 11, wobei die Behandlungsplandaten für die Strahlentherapiebehandlung einen Satz von Behandlungsverabreichungsparametern umfassen, die Fähigkeiten einer Strahlentherapiebehandlungsmaschine entsprechen;

und wobei optional die Strahlentherapiebehandlung mit einem Gammamesser zu versehen ist und der Satz von Behandlungsverabreichungsparametern einen Satz von Isozentren umfasst, die zur Verabreichung der Strahlentherapiebehandlung verwendet werden;
und wobei optional der Satz von Behandlungsverabreichungsparametern ferner eine Zeitsteuerung für die Verabreichung der Strahlentherapiebehandlung und eine Kollimatorsequenz für die Verabreichung der Strahlentherapiebehandlung umfasst;
und wobei optional die Strahlentherapiebehandlung mit einer volumenmodulierten Rotationsbestrahlung (VMAT) oder einer intensitätsmodulierten Strahlentherapie (IMRT) unter Verwendung einer Linac-Strahlentherapiemaschine versehen ist und wobei der Satz von Behandlungsverabreichungparametern Folgendes umfasst: einen Satz von Bogensteuerpunkten für einen oder mehrere Bögen, Fluenzfelder, Gantry-Geschwindigkeit und Dosisrate entlang der ein oder mehreren Bögen.

**13.** Computerlesbares Speichermedium, umfassend computerlesbare Anweisungen zur Strahlentherapiebehandlungsplanung, wobei die Anweisungen, wenn sie ausgeführt werden, eine Rechenmaschine veranlassen, ein beliebiges der Verfahren nach Anspruch 1 bis 12 durchzuführen.

**14.** Computersystem, das ausgelegt ist zur Strahlentherapiebehandlungsplanung, wobei das System Folgendes umfasst:

eine oder mehrere Parallelverarbeitungshardware-Vorrichtungen;
eine oder mehrere Speichervorrichtungen zum Speichern von Daten zum Bereitstellen einer Strahlentherapiebehandlung für ein menschliches Subjekt; und
einen oder mehrere Prozessoren, die dazu ausgelegt sind, die Verfahren nach Anspruch 1 bis 12 durchzuführen.

## Revendications

**1.** Procédé mis en œuvre par ordinateur pour la planification d'un traitement par radiothérapie, comprenant :

l'obtention d'un ensemble de premiers problèmes d'optimisation pour dispenser un traitement par radiothérapie à un sujet humain, les premiers problèmes d'optimisation étant définis par une première pluralité de paramètres ;
la réalisation d'une optimisation de dose pour l'administration du traitement par radiothérapie à au moins une zone de traitement du sujet humain, l'optimisation de dose comprenant :

la conversion des premiers problèmes d'optimisation en une première matrice de problèmes ;

la réalisation d'un premier passage de l'optimisation de dose par résolution, à l'aide d'un solveur de programmation linéaire fonctionnant selon la technique de la méthode des directions alternées des multiplicateurs, des premiers problèmes d'optimisation représentés dans la matrice de premiers problèmes en parallèle sur du matériel de traitement parallèle, le premier passage devant produire un premier ensemble de solutions multiples, correspondant à une première pluralité de plusieurs ensembles de poids, aux premiers problèmes d'optimisation ;
la combinaison du premier ensemble de solutions multiples et des premiers problèmes d'optimisation pour produire un ensemble de seconds problèmes d'optimisation pour dispenser le traitement par radiothérapie, les seconds problèmes d'optimisation étant définis par une seconde pluralité de paramètres ;
la conversion des seconds problèmes d'optimisation en une seconde matrice de problèmes ; et
la réalisation d'un second passage de l'optimisation de dose par résolution, à l'aide du solveur de programmation linéaire fonctionnant selon la technique de la méthode des directions alternées des multiplicateurs, des seconds problèmes d'optimisation représentés dans la seconde matrice de problèmes en parallèle sur le matériel de traitement parallèle, le second passage devant produire un second ensemble de solutions multiples, correspondant à une seconde pluralité de multiples ensembles de poids, aux seconds problèmes d'optimisation ; et
la génération de données de plan de traitement sur la base d'au moins une solution du second ensemble de solutions multiples aux seconds problèmes d'optimisation, les données de plan de traitement pouvant être utilisées pour contrôler l'administration de la radiothérapie à partir d'une machine de radiothérapie ; dans lequel l'au moins une zone de traitement comporte une région de faible dose et une région cible, dans lequel une dose à administrer dans la région de faible dose est une fraction d'une dose à administrer dans la région cible, et dans lequel la combinaison du premier ensemble de solutions multiples pour produire les seconds problèmes d'optimisation comprend :
la réalisation d'une union du premier ensemble de solutions multiples aux premiers problèmes d'optimisation pour la région de faible dose.

**2.** Procédé selon la revendication 1, dans lequel la première et la seconde pluralité de paramètres définissent des contraintes pour au moins une zone cible et au moins une zone de faible dose dans l'au moins une zone de traitement.

**3.** Procédé selon l'une quelconque des revendications 1 à 2, dans lequel les première et seconde pluralités d'ensembles multiples de poids, correspondant aux premier et second ensembles de solutions multiples, concernent des points définis pour au moins un volume de faible dose.

**4.** Procédé selon l'une quelconque des revendications 1 à 3, dans lequel la première pluralité de paramètres et la seconde pluralité de paramètres concernent des paramètres d'administration de rayonnement d'une machine de traitement par radiothérapie.

**5.** Procédé selon l'une quelconque des revendications 1 à 4, dans lequel la résolution des premiers problèmes d'optimisation ou des seconds problèmes d'optimisation en parallèle sur le matériel de traitement parallèle comprend, pour un ensemble de problèmes respectif :

l'identification d'équations de programmation linéaire paramétrées à partir de l'ensemble de problèmes respectif ; et
la conversion des équations de programmation linéaire paramétrées pour une exécution par le matériel de traitement parallèle ; et
dans lequel la résolution de l'ensemble de problèmes respectif en parallèle comprend la résolution d'une pluralité d'équations de programmation linéaire paramétrées converties en parallèle sur le matériel de traitement parallèle, pour produire une pluralité de solutions à l'ensemble de problèmes respectif.

**6.** Procédé selon la revendication 5, dans lequel la conversion des équations de programmation linéaire paramétrées comprend l'application de la technique de la méthode des directions alternées des multiplicateurs, et dans lequel la technique de la méthode des directions alternées des multiplicateurs comprend la transformation des équations de programmation linéaire paramétrées converties en opérations de matrice et de projection.

**7.** Procédé selon l'une quelconque des revendications 1 à 6, dans lequel le matériel de traitement parallèle comprend un ensemble d'une ou plusieurs unités de traitement graphique (GPU).

**8.** Procédé selon l'une quelconque des revendications 1 à 7, dans lequel chaque point de faible dose sélectionné à partir d'une région commune de faible dose du premier ensemble de solutions multiples des premiers problèmes d'optimisation est représenté dans la seconde matrice de problèmes, et dans lequel l'exécution du second passage de l'optimisation de dose comporte l'attribution d'une borne supérieure non nulle à un vecteur solution dans un sous-ensemble de points correspondant à une région de faible dose respective pour chaque ensemble de poids.

**9.** Procédé selon l'une quelconque des revendications 1 à 7, dans lequel chaque point de faible dose sélectionné dans une région commune de faible dose du premier ensemble de solutions multiples du premier problème d'optimisation est représenté dans la seconde matrice de problème, et dans lequel l'exécution du second passage de l'optimisation de dose comporte l'application d'un nouveau poids de faible dose à tous les points de faible dose dans l'union du premier ensemble de solutions multiples des premiers problèmes d'optimisation.

**10.** Procédé selon l'une quelconque des revendications 1 à 7, dans lequel la combinaison du premier ensemble de solutions multiples pour produire les seconds problèmes d'optimisation comprend :
la réalisation d'un échantillonnage de l'union du premier ensemble de solutions multiples pour identifier les poids de la seconde pluralité de paramètres pour la région de faible dose.

**11.** Procédé selon l'une quelconque des revendications 1 à 10, comprenant en outre :

la sélection d'une solution aux seconds problèmes d'optimisation sur la base d'une évaluation du second ensemble de solutions multiples, dans lequel les données du plan de traitement sont générées sur la base de la solution aux seconds problèmes d'optimisation sélectionnée ;
et éventuellement, dans lequel la solution aux seconds problèmes d'optimisation sélectionnée fournit une solution approximative, le procédé comprenant en outre la réception d'une optimisation supplémentaire à la solution sélectionnée, dans lequel les données du plan de traitement sont générées sur la base de l'optimisation supplémentaire de la solution sélectionnée.

**12.** Procédé selon l'une quelconque des revendications 1 à 11, dans lequel les données du plan de traitement pour le traitement par radiothérapie comprennent un ensemble de paramètres d'administration de traitement correspondant aux capacités d'une machine de traitement par radiothérapie ;

et éventuellement, dans lequel le traitement par radiothérapie doit être fourni avec un Gamma Knife, et l'ensemble de paramètres d'administration du traitement comprend un ensemble d'isocentres utilisés pour l'administration du traitement par radiothérapie ;
et éventuellement, dans lequel l'ensemble de paramètres d'administration de traitement comprend en outre une durée pour l'administration du traitement par radiothérapie et une séquence de collimateur pour l'administration du traitement par radiothérapie ;
et éventuellement, dans lequel le traitement par radiothérapie est réalisé au moyen d'une thérapie par arc modulé en intensité volumétrique (VMAT) ou d'une radiothérapie modulée en intensité (IMRT) au moyen d'un appareil de radiothérapie Linac, et dans lequel l'ensemble de paramètres d'administration de traitement comprend : un ensemble de points de contrôle d'arc pour un ou plusieurs arcs, des champs de fluence, la vitesse du portique et le débit de dose le long du ou des arcs.

**13.** Support de stockage lisible par ordinateur comprenant des instructions lisibles par ordinateur pour la planification d'un traitement par radiothérapie, dans lequel les instructions, lorsqu'elles sont exécutées, amènent une machine informatique à effectuer un quelconque des procédés selon les revendications 1 à 12.

**14.** Système informatique configuré pour la planification d'un traitement par radiothérapie, le système informatique comprenant :

un ou plusieurs dispositifs matériels de traitement parallèle ;
un ou plusieurs dispositifs de mémoire pour stocker des données pour fournir un traitement par radiothérapie à un sujet humain ; et
un ou plusieurs processeurs conçus pour effectuer l'un quelconque des procédés selon les revendications 1 à 12.

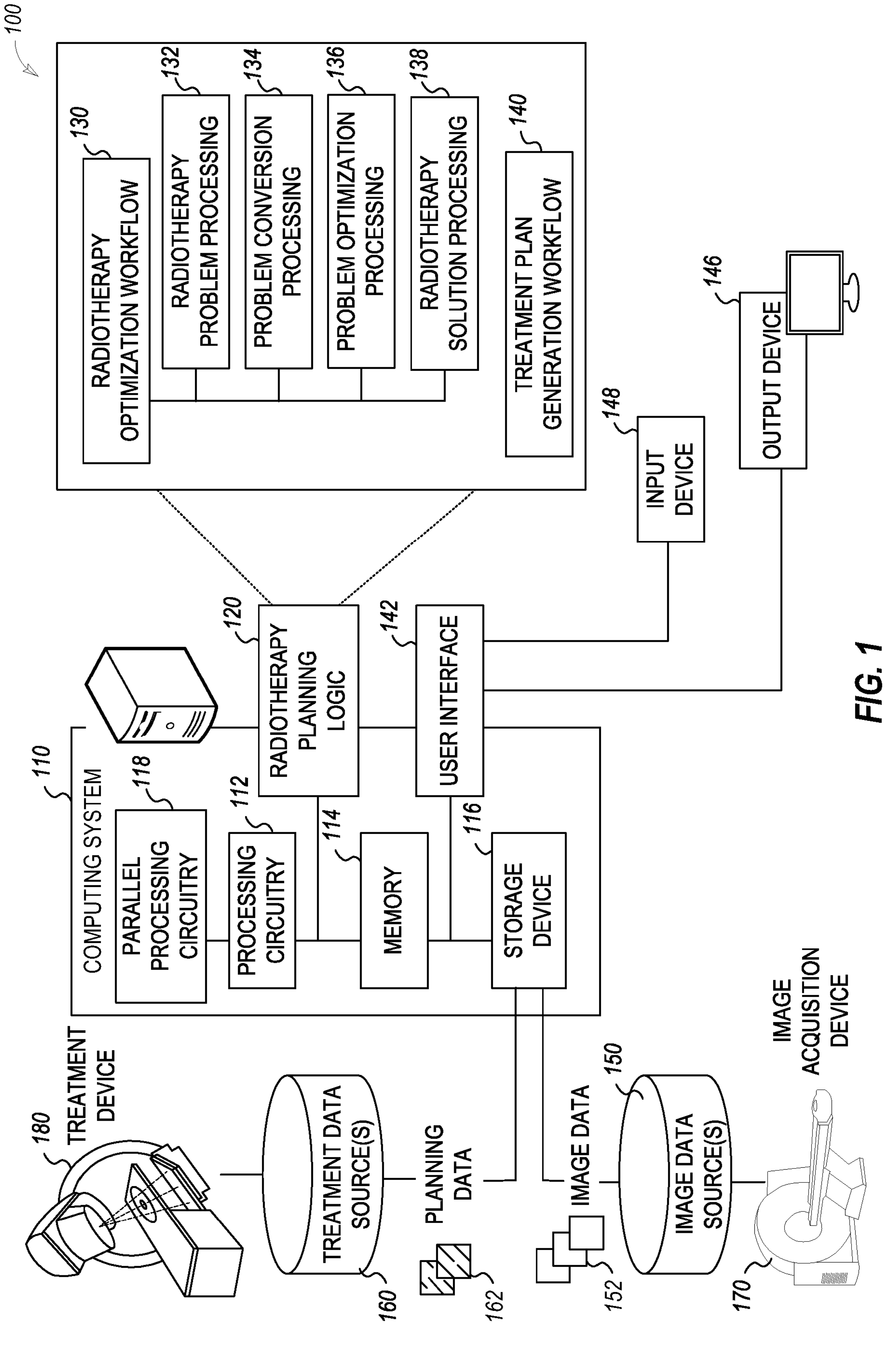
100
130
RADIOTHERAPY OPTIMIZATION WORKFLOW
132
RADIOTHERAPY PROBLEM PROCESSING
134
PROBLEM CONVERSION PROCESSING
136
PROBLEM OPTIMIZATION PROCESSING
138
RADIOTHERAPY SOLUTION PROCESSING
140
TREATMENT PLAN GENERATION WORKFLOW
148
INPUT DEVICE
146
OUTPUT DEVICE
110
COMPUTING SYSTEM
118
PARALLEL PROCESSING CIRCUITRY
112
PROCESSING CIRCUITRY
114
MEMORY
116
STORAGE DEVICE
120
RADIOTHERAPY PLANNING LOGIC
142
USER INTERFACE
180
TREATMENT DEVICE
160
TREATMENT DATA SOURCE(S)
PLANNING DATA
162
IMAGE DATA
152
150
IMAGE DATA SOURCE(S)
IMAGE ACQUISITION DEVICE
170

FIG. 1

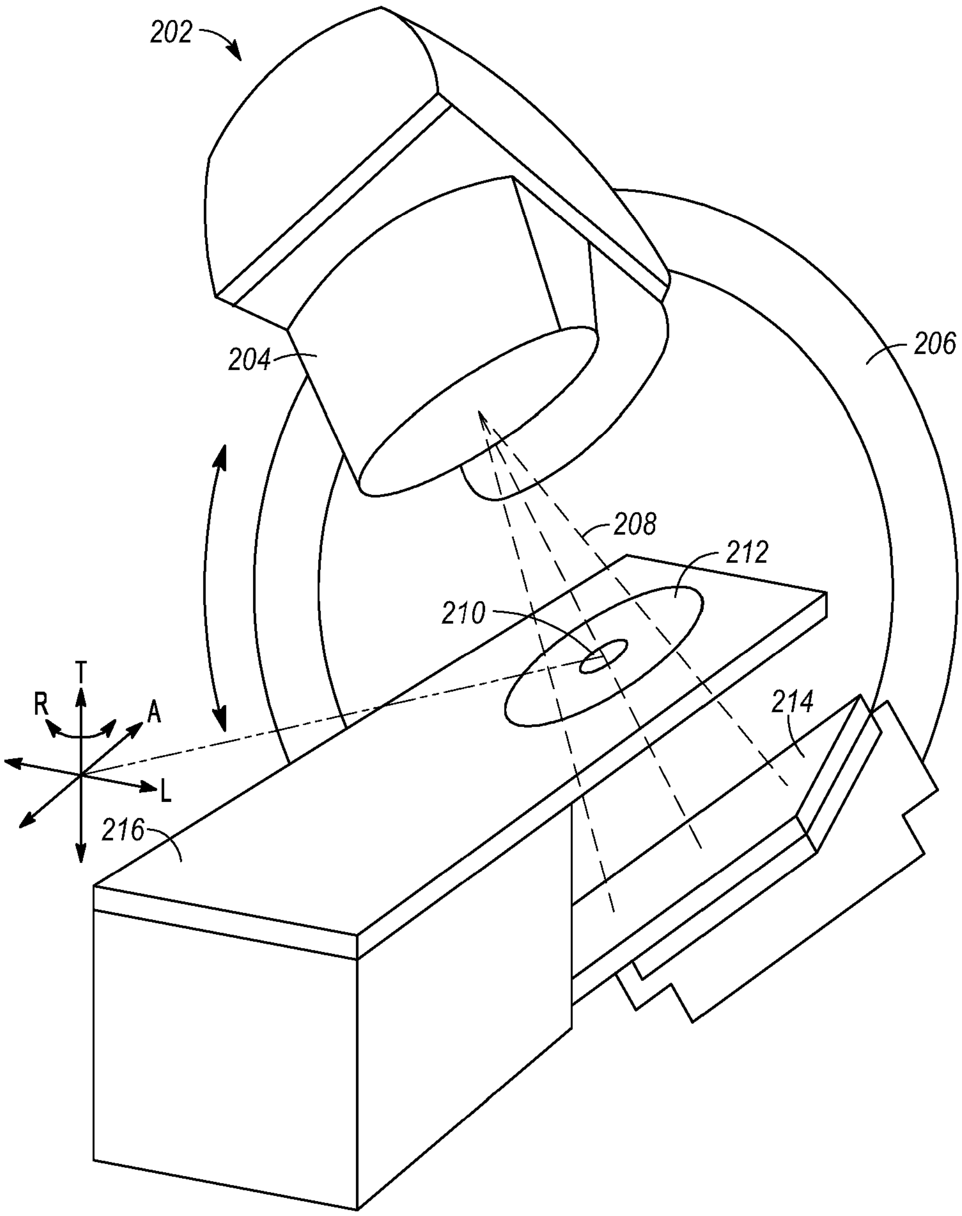


FIG. 2A

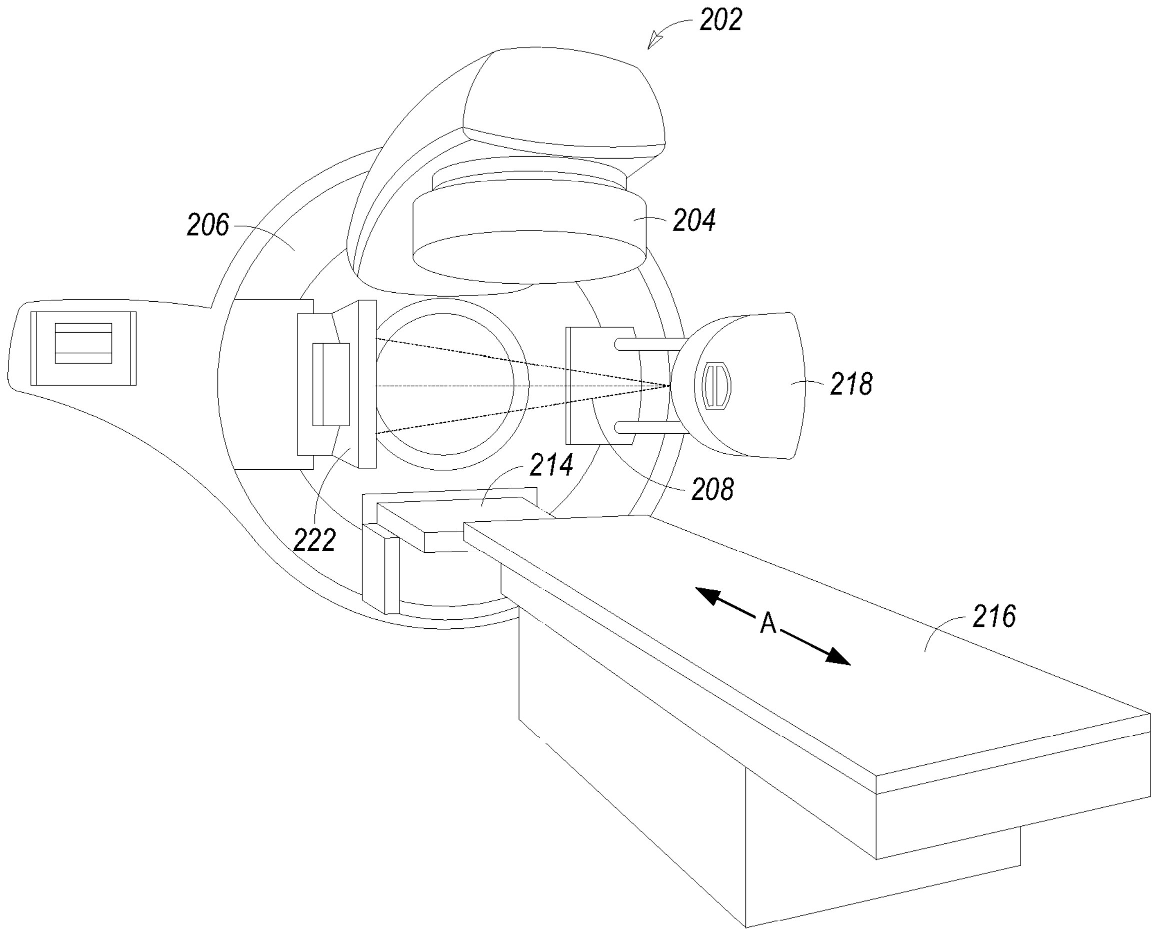


FIG. 2B

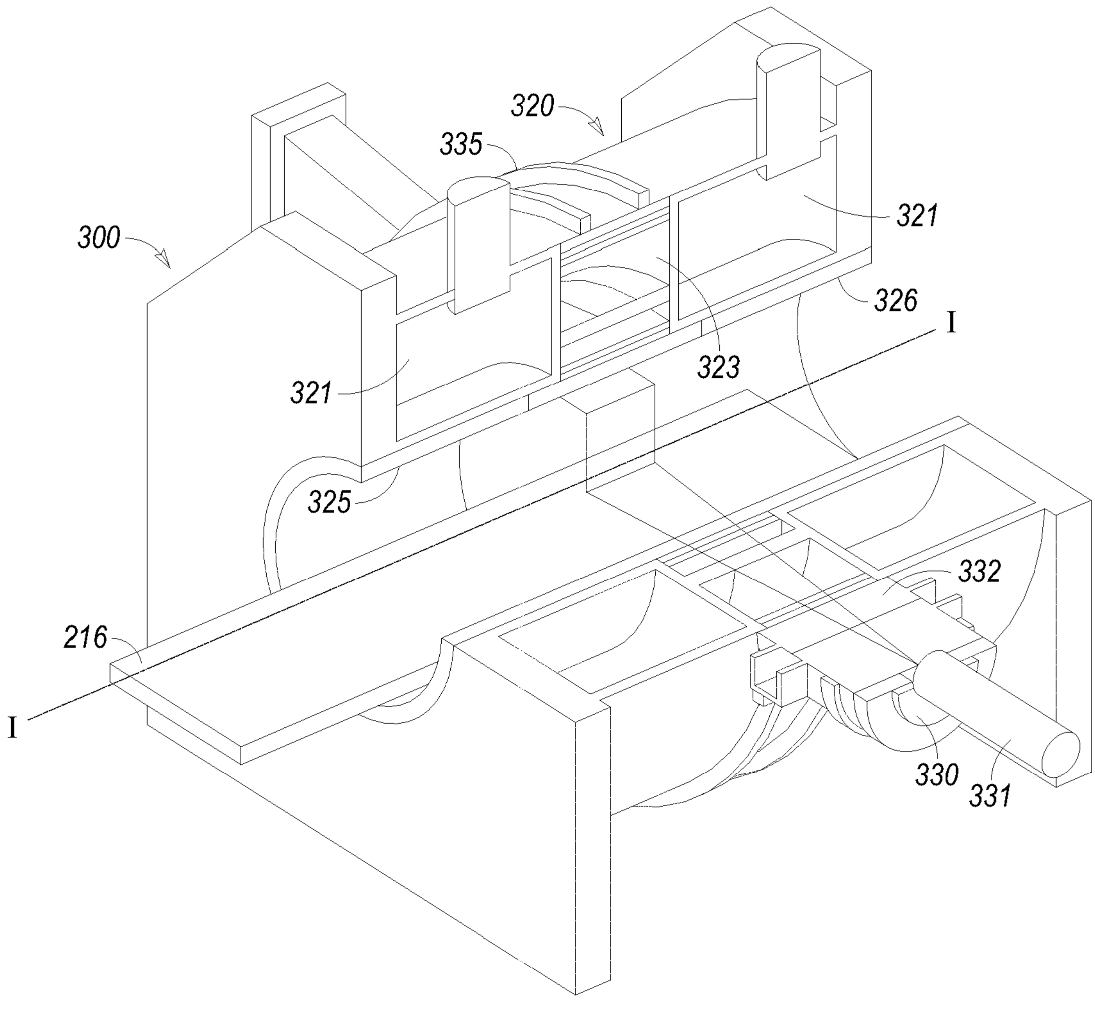


FIG. 3

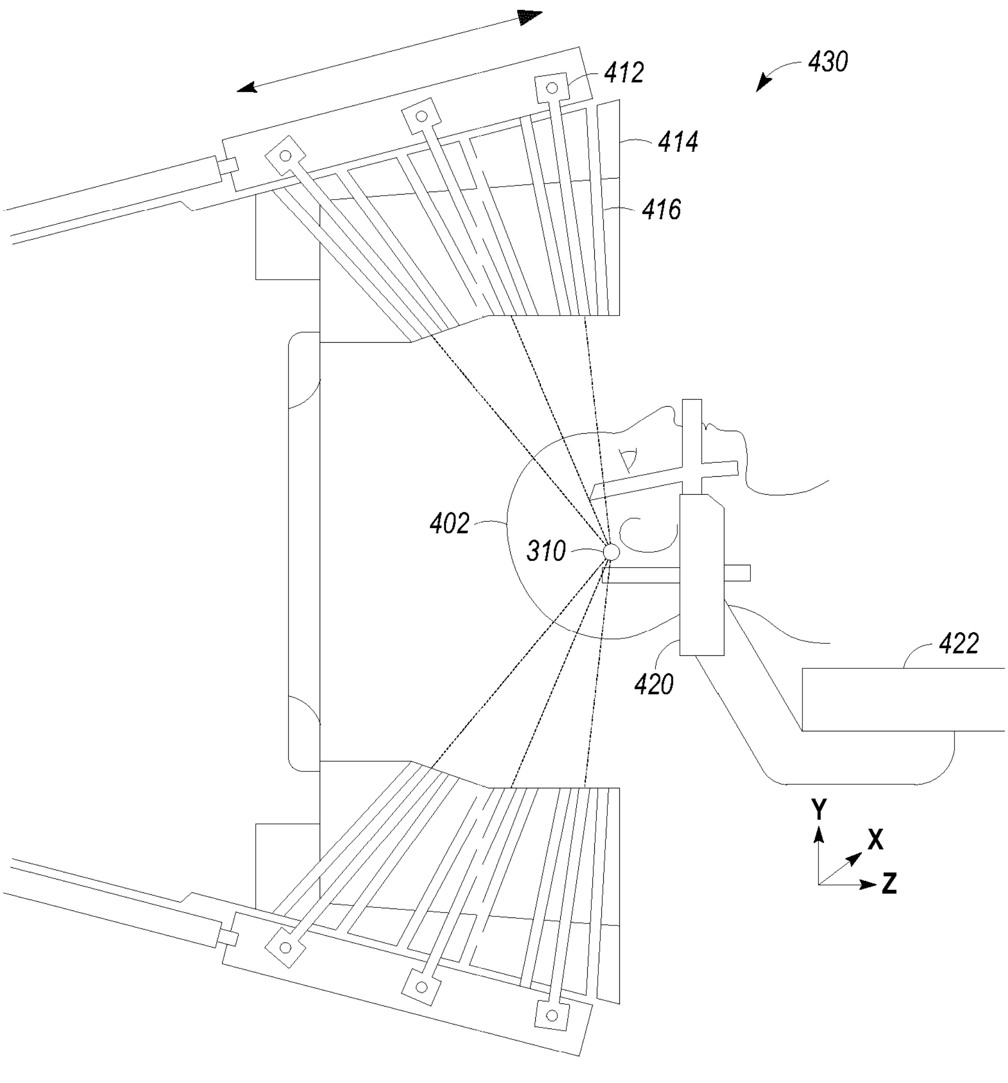


*FIG. 4*

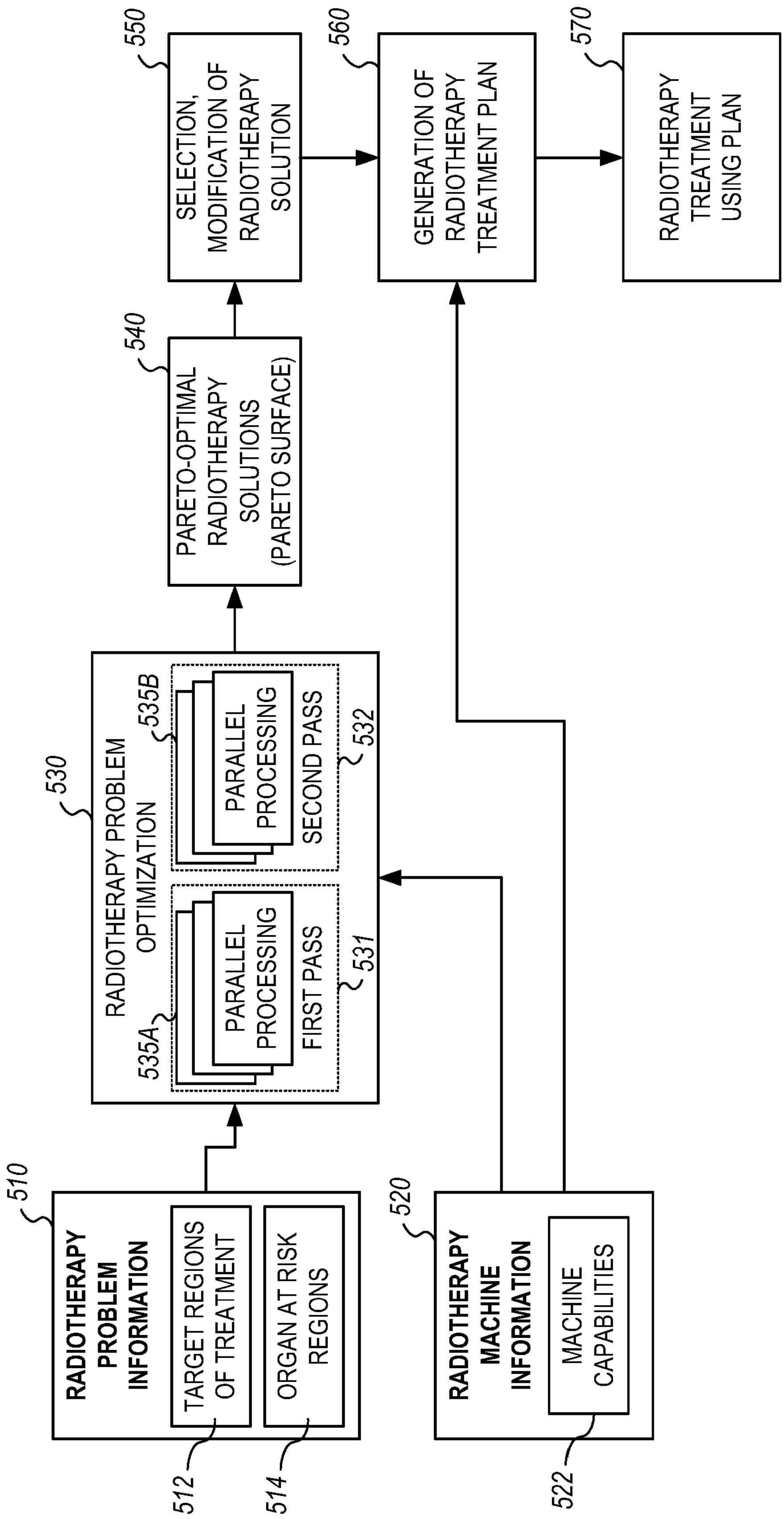
510
RADIOTHERAPY PROBLEM INFORMATION
TARGET REGIONS OF TREATMENT
ORGAN AT RISK REGIONS
512
514
520
RADIOTHERAPY MACHINE INFORMATION
MACHINE CAPABILITIES
522
530
RADIOTHERAPY PROBLEM OPTIMIZATION
535A
PARALLEL PROCESSING
FIRST PASS
531
535B
PARALLEL PROCESSING
SECOND PASS
532
540
PARETO-OPTIMAL RADIOTHERAPY SOLUTIONS (PARETO SURFACE)
550
SELECTION, MODIFICATION OF RADIOTHERAPY SOLUTION
560
GENERATION OF RADIOTHERAPY TREATMENT PLAN
570
RADIOTHERAPY TREATMENT USING PLAN

FIG. 5

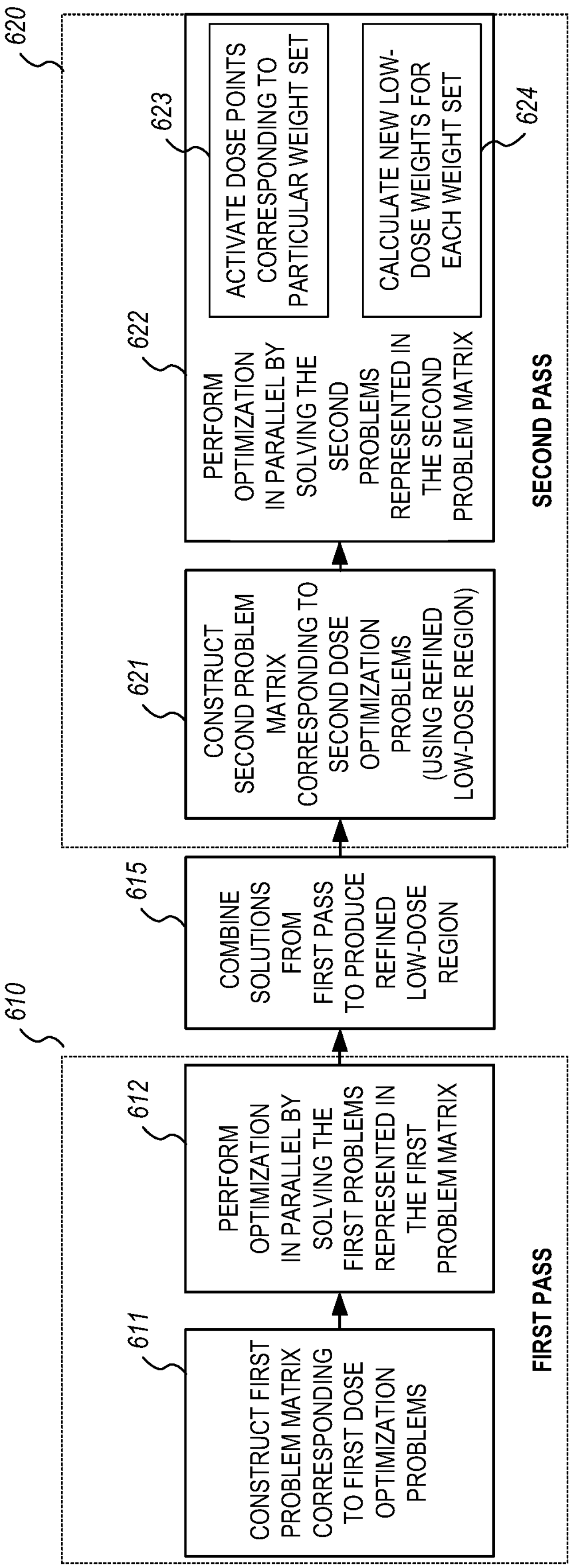
610
FIRST PASS
611
CONSTRUCT FIRST PROBLEM MATRIX CORRESPONDING TO FIRST DOSE OPTIMIZATION PROBLEMS
612
PERFORM OPTIMIZATION IN PARALLEL BY SOLVING THE FIRST PROBLEMS REPRESENTED IN THE FIRST PROBLEM MATRIX
615
COMBINE SOLUTIONS FROM FIRST PASS TO PRODUCE REFINED LOW-DOSE REGION
620
SECOND PASS
621
CONSTRUCT SECOND PROBLEM MATRIX CORRESPONDING TO SECOND DOSE OPTIMIZATION PROBLEMS (USING REFINED LOW-DOSE REGION)
622
PERFORM OPTIMIZATION IN PARALLEL BY SOLVING THE SECOND PROBLEMS REPRESENTED IN THE SECOND PROBLEM MATRIX
623
ACTIVATE DOSE POINTS CORRESPONDING TO PARTICULAR WEIGHT SET
624
CALCULATE NEW LOW-DOSE WEIGHTS FOR EACH WEIGHT SET

FIG. 6

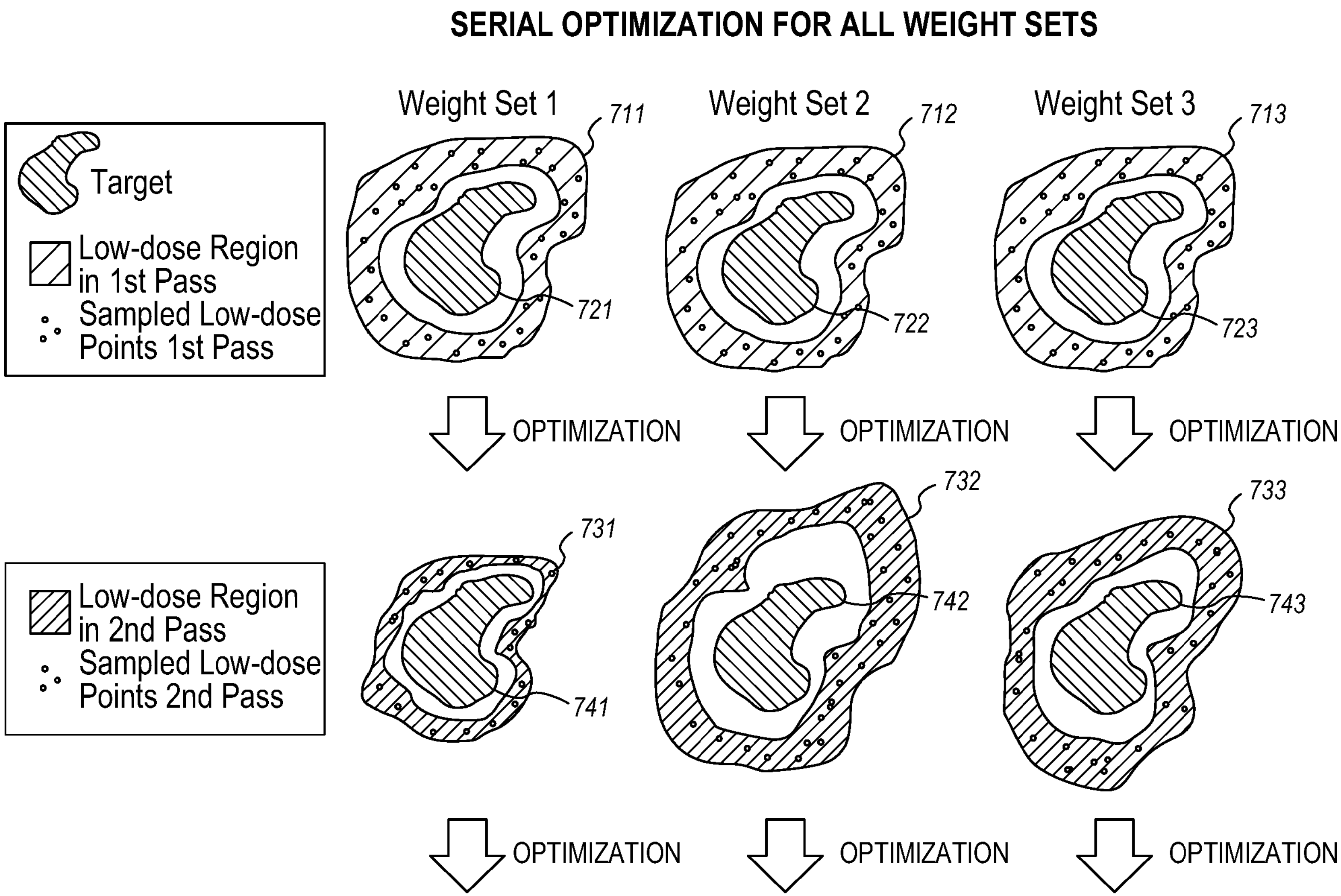
SERIAL OPTIMIZATION FOR ALL WEIGHT SETS
Target
Low-dose Region in 1st Pass
Sampled Low-dose Points 1st Pass
Low-dose Region in 2nd Pass
Sampled Low-dose Points 2nd Pass
Weight Set 1
Weight Set 2
Weight Set 3
711
712
713
721
722
723
OPTIMIZATION
OPTIMIZATION
OPTIMIZATION
731
732
733
741
742
743
OPTIMIZATION
OPTIMIZATION
OPTIMIZATION

FIG. 7A

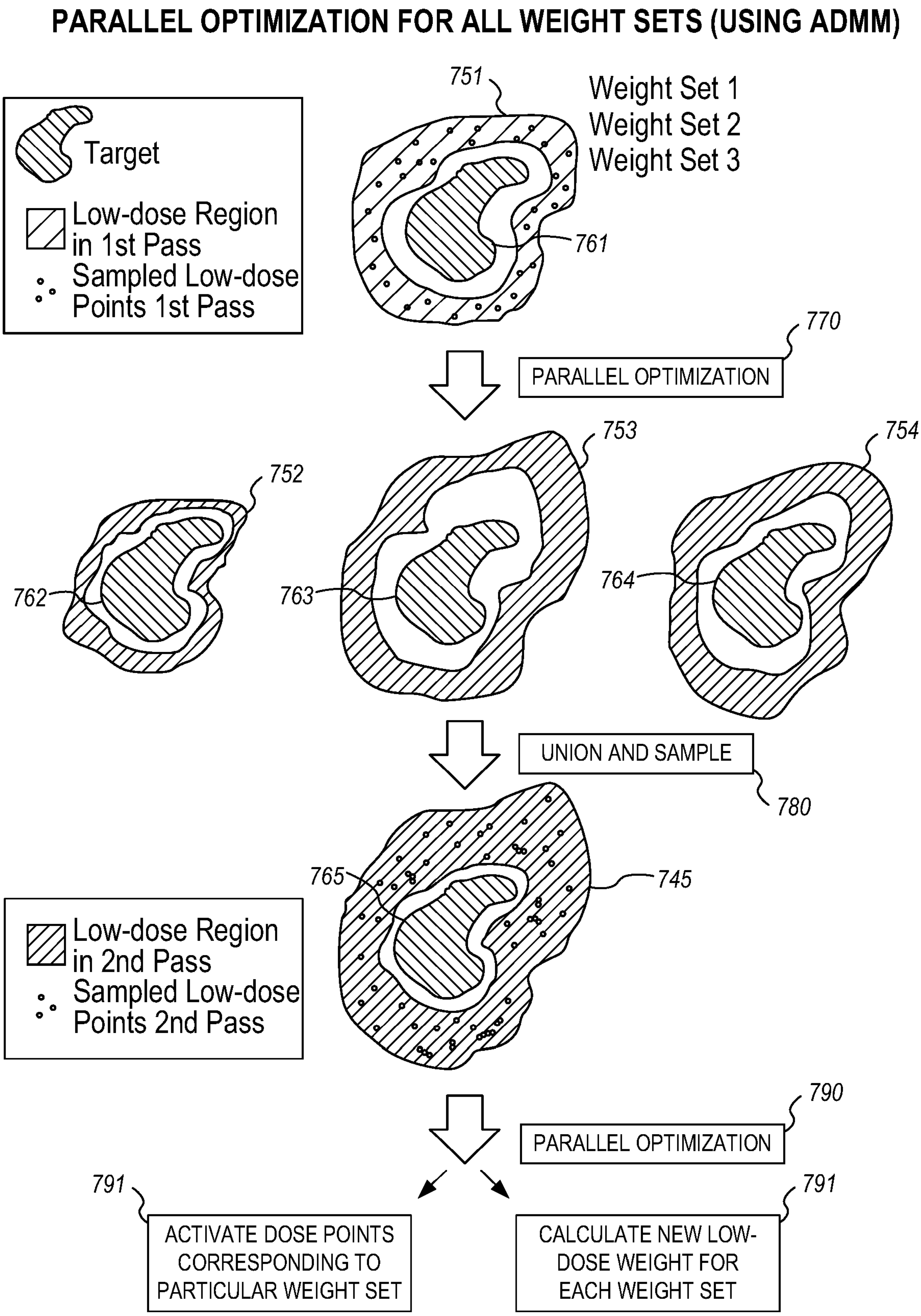
PARALLEL OPTIMIZATION FOR ALL WEIGHT SETS (USING ADMM)
Target
Low-dose Region in 1st Pass
Sampled Low-dose Points 1st Pass
751
Weight Set 1
Weight Set 2
Weight Set 3
761
770
PARALLEL OPTIMIZATION
752
753
754
762
763
764
UNION AND SAMPLE
780
765
745
Low-dose Region in 2nd Pass
Sampled Low-dose Points 2nd Pass
790
PARALLEL OPTIMIZATION
791
791
ACTIVATE DOSE POINTS CORRESPONDING TO PARTICULAR WEIGHT SET
CALCULATE NEW LOW-DOSE WEIGHT FOR EACH WEIGHT SET

*FIG. 7B*

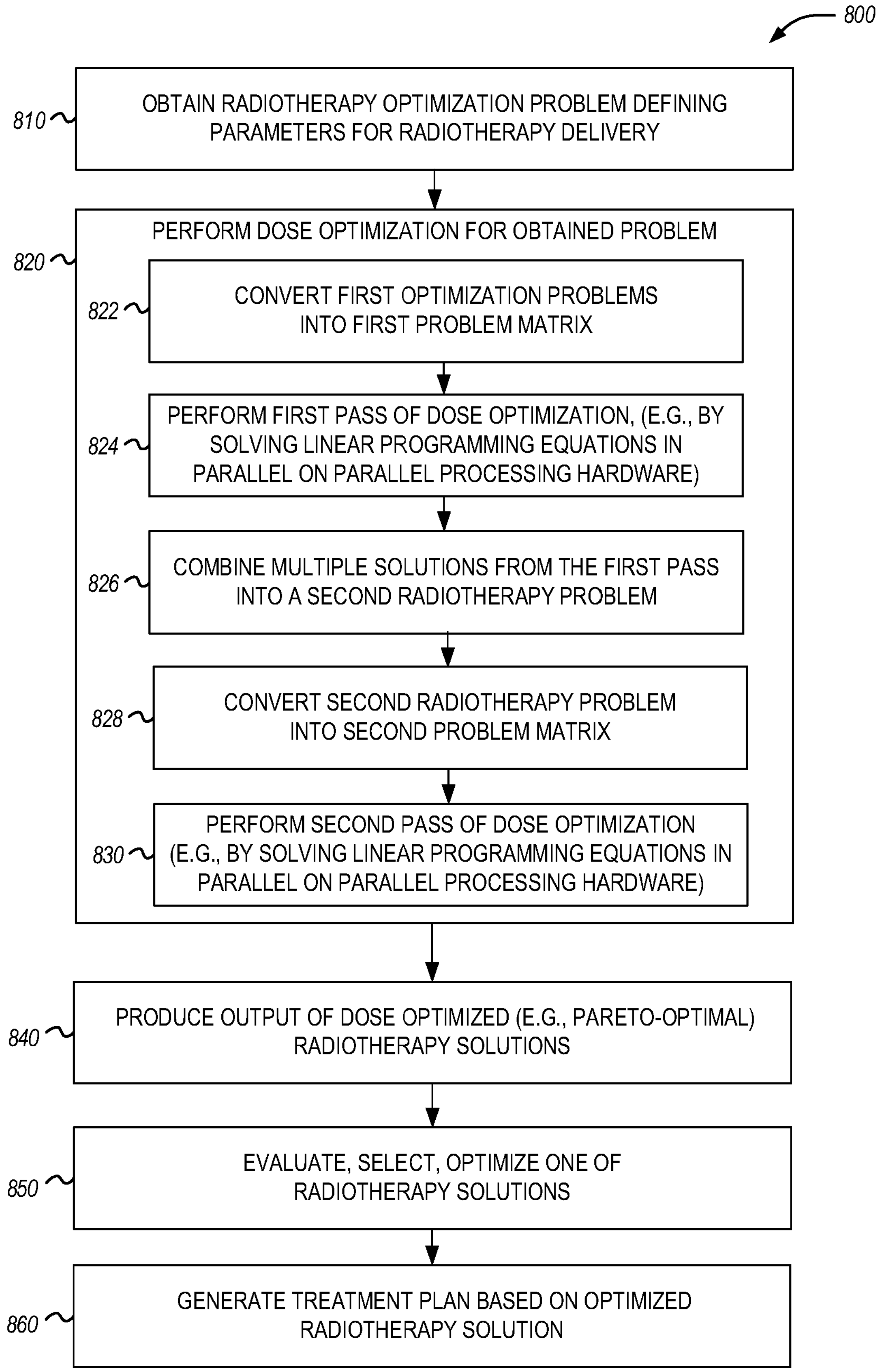
800
810
OBTAIN RADIOTHERAPY OPTIMIZATION PROBLEM DEFINING PARAMETERS FOR RADIOTHERAPY DELIVERY
820
PERFORM DOSE OPTIMIZATION FOR OBTAINED PROBLEM
822
CONVERT FIRST OPTIMIZATION PROBLEMS INTO FIRST PROBLEM MATRIX
824
PERFORM FIRST PASS OF DOSE OPTIMIZATION, (E.G., BY SOLVING LINEAR PROGRAMMING EQUATIONS IN PARALLEL ON PARALLEL PROCESSING HARDWARE)
826
COMBINE MULTIPLE SOLUTIONS FROM THE FIRST PASS INTO A SECOND RADIOTHERAPY PROBLEM
828
CONVERT SECOND RADIOTHERAPY PROBLEM INTO SECOND PROBLEM MATRIX
830
PERFORM SECOND PASS OF DOSE OPTIMIZATION (E.G., BY SOLVING LINEAR PROGRAMMING EQUATIONS IN PARALLEL ON PARALLEL PROCESSING HARDWARE)
840
PRODUCE OUTPUT OF DOSE OPTIMIZED (E.G., PARETO-OPTIMAL) RADIOTHERAPY SOLUTIONS
850
EVALUATE, SELECT, OPTIMIZE ONE OF RADIOTHERAPY SOLUTIONS
860
GENERATE TREATMENT PLAN BASED ON OPTIMIZED RADIOTHERAPY SOLUTION

FIG. 8

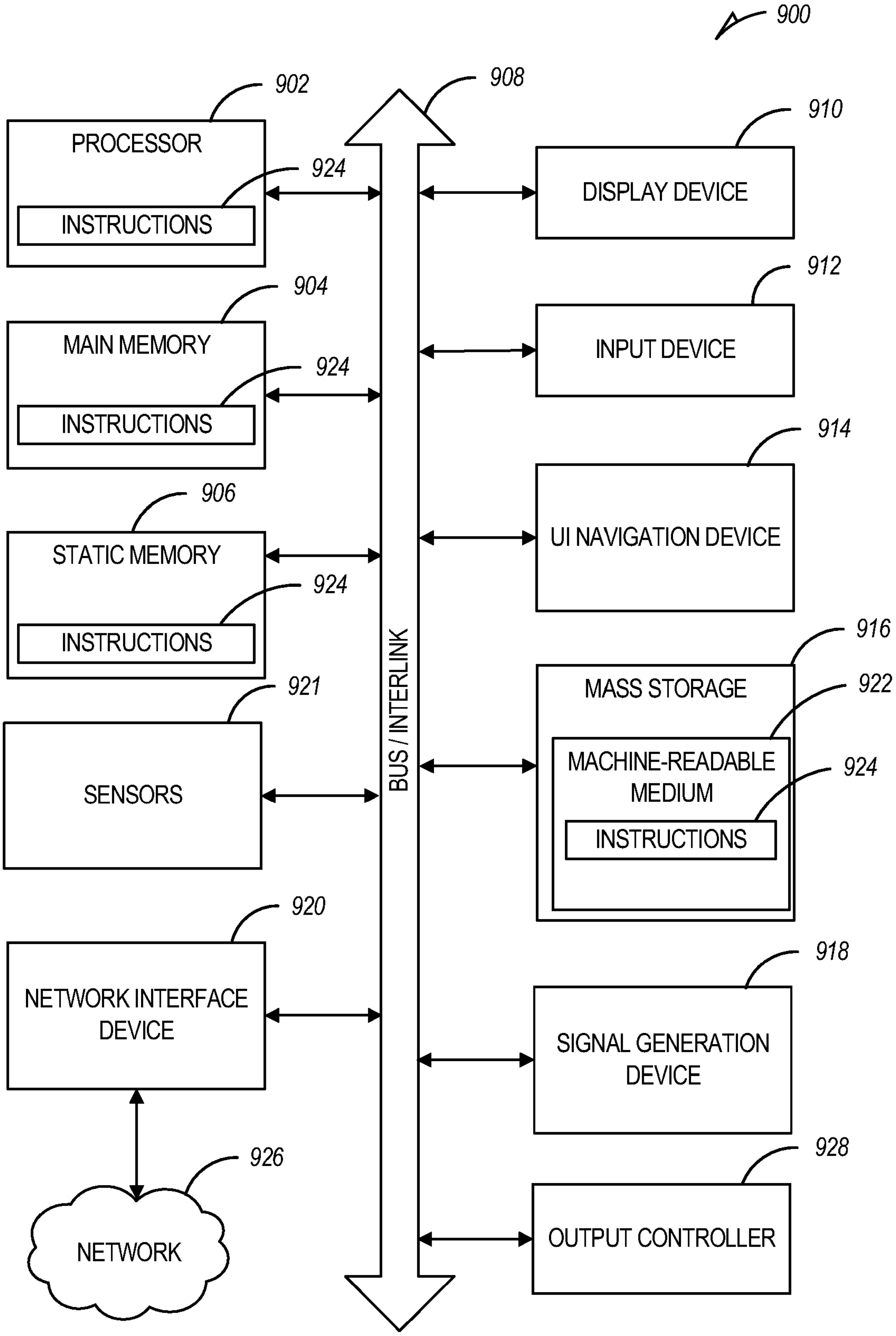
900
902
PROCESSOR
INSTRUCTIONS
924
908
910
DISPLAY DEVICE
904
MAIN MEMORY
INSTRUCTIONS
924
912
INPUT DEVICE
914
UI NAVIGATION DEVICE
906
STATIC MEMORY
INSTRUCTIONS
924
BUS / INTERLINK
916
MASS STORAGE
922
MACHINE-READABLE MEDIUM
924
INSTRUCTIONS
921
SENSORS
920
NETWORK INTERFACE DEVICE
918
SIGNAL GENERATION DEVICE
926
NETWORK
928
OUTPUT CONTROLLER

*FIG. 9*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2021158929 A1
- EP 2022053438 W
- WO 2023151816 A1
- EP 2022053440 W
- WO 2023151817 A1

### Non-patent literature cited in the description


- **BOYD et al.** Distributed Optimization and Statistical Learning via the Alternating Direction Method of Multipliers. Now Publishers Inc., 2011
- **NAIR et al.** Solving Mixed Integer Programs Using Neural Networks. *arXiv:2012.13349*, 2020